# EUROPEAN PATENT APPLICATION

(11) **EP 4 740 860 A1**
(43) Date of publication of application: **13.05.2026**
(21) Application number: 24834235.4
(22) Date of filing: 08.07.2024
(51) Int. Cl.: A61B 5/16, A61B 5/374, A61B 5/397

(54) **INFORMATION PROCESSING DEVICE, SLEEP/WAKEFULNESS STAGE DETERMINATION DEVICE, COMPUTER PROGRAM, INFORMATION PROCESSING METHOD, AND SLEEP/WAKEFULNESS STAGE DETERMINATION METHOD**

(30) Priority: 07.07.2023 JP 2023111974
(71) Applicant: University of Tsukuba, Ibaraki 305-8577 (JP)
(72) Inventor: SAKURAI, Takeshi, Tsukuba-shi, Ibaraki 305-8577 (JP); FURUTANI, Naoki, Kanazawa-shi, Ishikawa 920-1192 (JP); SAITO, Yuki, Tsukuba-shi, Ibaraki 305-8577 (JP)
(74) Representative: Gill Jennings & Every LLP
(86) International application number: PCT/JP2024/024606
(87) International publication number: WO 2025/013824

(57) **Abstract**

Included are an electroencephalogram processing unit that obtains an amplitude and a phase from training electroencephalogram data measured from a living body, which is a mammal, for each of a predetermined plurality of frequency bands, and performs complexity analysis processing comprising entropy analysis on each of the amplitude and the phase obtained in each of the plurality of frequency bands, and a machine learning unit that performs machine learning processing of a machine learning model that determines sleep/wakefulness stages using a result of the complexity analysis processing and label data of the sleep/wakefulness stages corresponding to the training electroencephalogram data.

## Description

### Technical Field

The present disclosure relates to an information processing device, a sleep/wakefulness stages determination device, a computer program, an information processing method, and a sleep/wakefulness stages determination method.

The present application claims priority based on Japanese Patent Application No. 2023-111974 filed in Japan on July 7, 2023, the contents of which are incorporated herein by reference.

### Background Art

In the past, in sleep research, experiments using mice have been conducted to determine sleep/wakefulness stages (wakefulness, non-REM sleep, REM sleep). As an automated technique for determining sleep/wakefulness stages, a technique that automatically determines sleep/wakefulness stages from mouse electroencephalogram data or electromyogram data is known (see, for example, NPTL 1). Known automated sleep/wakefulness stages determination techniques determine sleep/wakefulness stages based on frequency spectra of electroencephalogram data or amplitudes of electromyogram data.

NPTL 2 describes that a combination of decomposition entropy, which is entropy decomposed into frequency band, phase, and amplitude using sample entropy (SampEn) of an electroencephalogram, and deep learning, is useful for diagnosis of Alzheimer's disease.

NPTL 3 describes that a combination of decomposition entropy using expanded sample entropy (expSampEn) of an electroencephalogram and deep learning is useful for neural decoding (such as predicting what a person thinks). The expanded sample entropy is calculated using an autocorrelation of a single point to all points in a time series.

NPTL 4 describes that expanded sample entropy of body movements (trunk acceleration) during night is useful for diagnosing autism spectrum disorder. In NPTL 4, decomposition entropy and machine learning/deep learning are not used.

### Citation List

### Non-Patent Literature

NPTL 1: Masato Yamabe et al., "MC-SleepNet: Large-scale Sleep Stage Scoring in Mice by Deep Neural Networks", SCIENTIFIC REPORTS, 9:15793, 2019
NPTL 2: Naoki Furutani et al., "Decomposed Temporal Complexity Analysis of Neural Oscillations and Machine Learning Applied to Alzheimer's Disease Diagnosis", Front. Psychiatry, 11:531801, 03 September 2020
NPTL 3: Naoki Furutani et al., "Neural Decoding of Multi-Modal Imagery Behavior Focusing on Temporal Complexity", Front. Psychiatry, 11:746, 30 July 2020
NPTL 4: Naoki Furutani et al., "Complexity of Body Movements during Sleep in Children with Autism Spectrum Disorder", Entropy, 23(4):418, 31 March 2021
NPTL 5: Dorde Miladinovic et al., "SPINDLE: End-to-end learning from EEG/EMG to extrapolate animal sleep scoring across experimental settings, labs and species", PLOS Computational Biology, 15(4):e1006968, 18 April 2019

### Summary of Invention

### Technical Problem

However, with the known automated sleep/wakefulness stages determination techniques described above, it has been difficult to improve accuracy of determination. NPTLs 2, 3, and 4 do not disclose anything regarding the determination of the sleep/wakefulness stages.

The present disclosure has been made in view of such circumstances, and an object thereof is to provide a technique that contributes to improvement of accuracy of automated determination of sleep/wakefulness stages.

### Solution to Problem

According to an aspect of the present disclosure, an information processing device includes an electroencephalogram processing unit configured to obtain an amplitude and a phase from training electroencephalogram data measured from a living body being a mammal for a plurality of frequency bands according to individual frequency bands, the plurality of frequency bands being predetermined, and perform complexity analysis processing comprising entropy analysis on each of the amplitude and the phase obtained in each of the plurality of frequency bands, and a machine learning unit configured to perform machine learning processing of a machine learning model configured to determine sleep/wakefulness stages using a result of the complexity analysis processing and label data of the sleep/wakefulness stages corresponding to the training electroencephalogram data.

According to an aspect of the present disclosure, in the information processing device, the electroencephalogram processing unit further extracts signals in the plurality of frequency bands, separately, from the training electroencephalogram data, and performs complexity analysis processing comprising entropy analysis on each of the signals extracted in each of the plurality of frequency bands, and the machine learning unit performs machine learning processing of the machine learning model by further using a result of the complexity analysis processing.

According to an aspect of the present disclosure, in the information processing device, the entropy analysis on the phase calculates an entropy of a cosine of the phase by performing entropy analysis on the cosine of the phase.

According to an aspect of the present disclosure, in the information processing device, the electroencephalogram processing unit uses expanded sample entropy as entropy analysis.

According to an aspect of the present disclosure, in the information processing device, when generating learning data used for machine learning of the machine learning model, the machine learning unit expands the learning data by standardization or normalization using a plurality of thresholds, or by both standardization and normalization using a plurality of thresholds.

According to an aspect of the present disclosure, in the information processing device, the living body is a mouse.

According to an aspect of the present disclosure, a sleep/wakefulness stages determination device includes an electroencephalogram processing unit configured to obtain an amplitude and a phase from electroencephalogram data measured from a living body being a mammal for a plurality of frequency bands according to individual frequency bands, the plurality of frequency bands being predetermined, and perform complexity analysis processing comprising entropy analysis on each of the amplitude and the phase obtained in each of the plurality of frequency bands, and a determination unit configured to determine sleep/wakefulness stages from a result of the complexity analysis processing using a determination model.

According to an aspect of the present disclosure, in the sleep/wakefulness stages determination device, the electroencephalogram processing unit further extracts signals in the plurality of frequency bands, separately, from the electroencephalogram data, and performs complexity analysis processing comprising entropy analysis on each of the signals extracted in each of the plurality of frequency bands, and the determination unit determines the sleep/wakefulness stages by further using a result of the complexity analysis processing.

According to an aspect of the present disclosure, in the sleep/wakefulness stages determination device, the entropy analysis on the phase calculates an entropy of a cosine of the phase by performing entropy analysis on the cosine of the phase.

According to an aspect of the present disclosure, in the sleep/wakefulness stages determination device, the electroencephalogram processing unit uses expanded sample entropy as entropy analysis.

According to an aspect of the present disclosure, the sleep/wakefulness stages determination device further includes a rescoring short epoch (RSE) method execution unit configured to apply an RSE method to determination results of the sleep/wakefulness stages determined using the determination model, to eliminate epochs having short stage durations in the determination results of the sleep/wakefulness stages by lowering priority of epochs determined to have stage durations shorter than a certain time, starting from an epoch having the shortest stage duration.

According to an aspect of the present disclosure, the sleep/wakefulness stages determination device further includes a skipping unit configured to suppress display of an epoch with a sufficiently high degree of confidence in the determination results of the sleep/wakefulness stages determined using the determination model.

According to an aspect of the present disclosure, the sleep/wakefulness stages determination device further includes an accuracy display control unit configured to display accuracy of determination for the determination results of the sleep/wakefulness stages determined using the determination model.

According to an aspect of the present disclosure, in the sleep/wakefulness stages determination device, the living body is a mouse.

According to an aspect of the present disclosure, a computer program is used for causing a computer to execute obtaining an amplitude and a phase from training electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands, and performing machine learning processing of a machine learning model for determination of sleep/wakefulness stages using a result of the complexity analysis processing and label data of the sleep/wakefulness stages corresponding to the training electroencephalogram data.

According to an aspect of the present disclosure, a computer program is used for causing a computer to execute obtaining an amplitude and a phase from electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands, and determining sleep/wakefulness stages from a result of the complexity analysis processing using a determination model.

According to an aspect of the present disclosure, an information processing method executed by an information processing device includes obtaining an amplitude and a phase from training electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands, and performing machine learning processing of a machine learning model for determination of sleep/wakefulness stages using a result of the complexity analysis processing and label data of the sleep/wakefulness stages corresponding to the training electroencephalogram data.

According to an aspect of the present disclosure, a sleep/wakefulness stages determination method executed by a sleep/wakefulness stages determination device includes obtaining an amplitude and a phase from electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands, and determining sleep/wakefulness stages from a result of the complexity analysis processing using a determination model.

### Advantageous Effects of Invention

According to the present disclosure, an effect of providing a technique that contributes to improvement of accuracy of automated determination of sleep/wakefulness stages can be obtained.

### Brief Description of Drawings

FIG. 1 is a block diagram illustrating a configuration example of an information processing device according to a first embodiment.
FIG. 2 is a block diagram illustrating a configuration example of a sleep/wakefulness stages determination device according to the first embodiment.
FIG. 3 is a block diagram illustrating a configuration example of an information processing device according to a second embodiment.
FIG. 4 is a block diagram illustrating a configuration example of a sleep/wakefulness stages determination device according to the second embodiment.
FIG. 5 is a block diagram illustrating a configuration example of an information processing device according to a third embodiment.
FIG. 6 is a block diagram illustrating a configuration example of a sleep/wakefulness stages determination device according to the third embodiment.
FIG. 7 is a block diagram illustrating a configuration example of an information processing device according to a fourth embodiment.
FIG. 8 is a block diagram illustrating a configuration example of a sleep/wakefulness stages determination device according to the fourth embodiment.
FIG. 9 is a block diagram illustrating a configuration example of an information processing device according to a fifth embodiment.
FIG. 10 is a block diagram illustrating a configuration example of a sleep/wakefulness stages determination device according to the fifth embodiment.
FIG. 11 is a table showing evaluation results according to the present disclosure.
FIG. 12 is a table showing evaluation results according to the present disclosure.
FIG. 13 is a table showing evaluation results according to the present disclosure.

### Description of Embodiments

Embodiments of the present disclosure will be described with reference to the drawings below. In the embodiments, sleep/wakefulness stages are determined for a living body that is a mammal. Examples of mammals include humans, monkeys, and mice. Hereinafter, a mouse will be used as an example of the mammal for explanation.

### First Embodiment

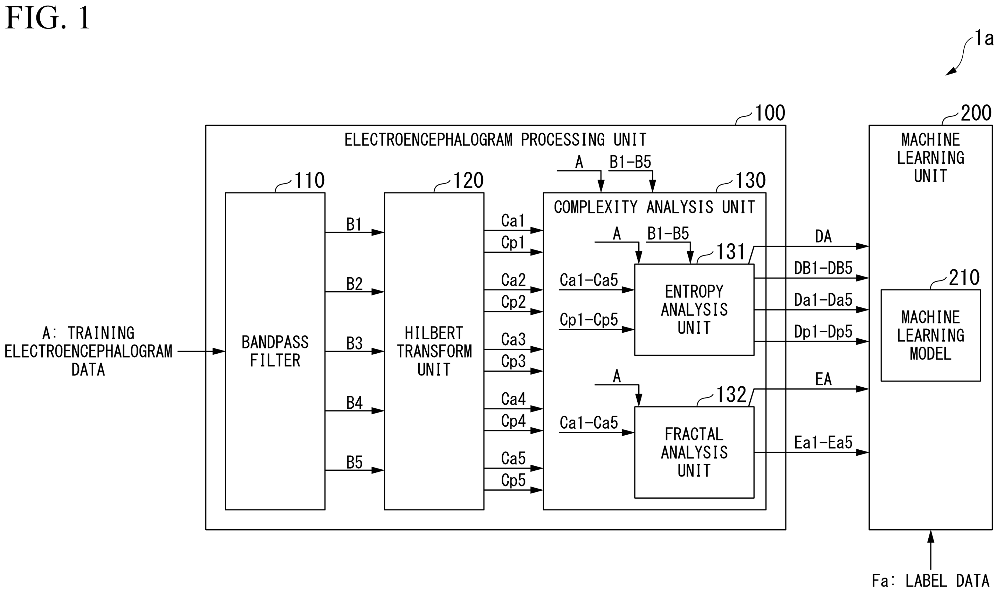

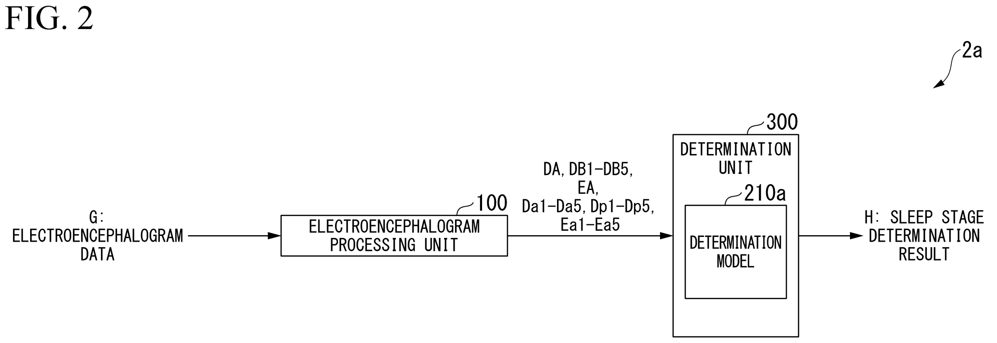

A first embodiment will be described with reference to FIGS. 1 and 2. FIG. 1 is a block diagram illustrating a configuration example of an information processing device according to the first embodiment. FIG. 2 is a block diagram illustrating a configuration example of a sleep/wakefulness stages determination device according to the first embodiment.

An information processing device 1a illustrated in FIG. 1 generates a determination model 210a used in a sleep/wakefulness stages determination device 2a illustrated in FIG. 2 by performing machine learning on a machine learning model 210 to determine sleep/wakefulness stages (wakefulness, non-REM sleep, REM sleep). No specific machine learning algorithm is required. As a machine learning algorithm, for example, deep learning may be used, or a light gradient boosting machine (LightGBM) may be used. The sleep/wakefulness stages determination device 2a illustrated in FIG. 2 determines the sleep/wakefulness stages using the determination model 210a generated by the information processing device 1a.

### Information Processing Device

The information processing device 1a according to the first embodiment will be described with reference to FIG. 1. Training electroencephalogram data A is input to the information processing device 1a. The training electroencephalogram data A is electroencephalogram time-series data measured from one mouse (living body), and is used for machine learning of the machine learning model 210. A plurality of training electroencephalogram data A may be used for machine learning of the machine learning model 210. For example, a plurality of electroencephalogram data measured from a plurality of mice (living bodies) may each be used as the training electroencephalogram data A.

The information processing device 1a includes an electroencephalogram processing unit 100 and a machine learning unit 200. The electroencephalogram processing unit 100 includes a bandpass filter 110, a Hilbert transform unit 120, and a complexity analysis unit 130. The complexity analysis unit 130 comprises an entropy analysis unit 131 and a fractal analysis unit 132.

The electroencephalogram processing unit 100 divides the training electroencephalogram data A into fixed intervals (epochs) in time series, and performs processing in each epoch. As one example in the present embodiment, each epoch is 25 seconds.

The bandpass filter 110 extracts signals in a predetermined plurality of frequency bands from one epoch of the training electroencephalogram data A. In the present embodiment, the bandpass filter 110 extracts, from one epoch of the training electroencephalogram data A, signals in a total of five frequency bands as an example of the plurality of frequency bands, namely, a delta wave band, a theta wave band, an alpha wave band, a beta wave band, and a gamma wave band (a delta wave band signal B1, a theta wave band signal B2, an alpha wave band signal B3, a beta wave band signal B4, and a gamma wave band signal B5). Therefore, the bandpass filter 110 comprises bandpass filters corresponding to the delta wave band, the theta wave band, the alpha wave band, the beta wave band, and the gamma wave band, respectively.

The Hilbert transform unit 120 performs a Hilbert transform on each of the delta wave band signal B1, the theta wave band signal B2, the alpha wave band signal B3, the beta wave band signal B4, and the gamma wave band signal B5. The Hilbert transform unit 120 obtains, for the delta wave band signal B1, the theta wave band signal B2, the alpha wave band signal B3, the beta wave band signal B4, and the gamma wave band signal B5 through the Hilbert transform, amplitudes "A(t)" (a delta wave band signal amplitude Ca1, a theta wave band signal amplitude Ca2, an alpha wave band signal amplitude Ca3, a beta wave band signal amplitude Ca4, and a gamma wave band signal amplitude Ca5) and phases "θ(t)" (a delta wave band signal phase Cp1, a theta wave band signal phase Cp2, an alpha wave band signal phase Cp3, a beta wave band signal phase Cp4, and a gamma wave band signal phase Cp5).

The complexity analysis unit 130 performs complexity analysis processing on each of the training electroencephalogram data A and the delta wave band signal amplitude Ca1, the theta wave band signal amplitude Ca2, the alpha wave band signal amplitude Ca3, the beta wave band signal amplitude Ca4, and the gamma wave band signal amplitude Ca5. The complexity analysis processing comprises entropy analysis by the entropy analysis unit 131 and fractal analysis by the fractal analysis unit 132.

The complexity analysis unit 130 performs complexity analysis processing on each of the delta wave band signal B1, the theta wave band signal B2, the alpha wave band signal B3, the beta wave band signal B4, and the gamma wave band signal B5, and on each of the delta wave band signal phase Cp1, the theta wave band signal phase Cp2, the alpha wave band signal phase Cp3, the beta wave band signal phase Cp4, and the gamma wave band signal phase Cp5. The complexity analysis processing comprises entropy analysis by the entropy analysis unit 131.

The entropy analysis calculates a value that indicates a degree of irregularity in a time series (hereinafter referred to as entropy) for a subject on which the entropy analysis is performed.

The fractal analysis calculates a value that indicates a degree of self-similarity in a time series (hereinafter referred to as fractal) for a subject on which the fractal analysis is performed.

The entropy analysis unit 131 performs entropy analysis on the training electroencephalogram data A. The entropy analysis unit 131 obtains an entropy of the training electroencephalogram data A (an electroencephalogram entropy DA) through this entropy analysis.

The entropy analysis unit 131 performs entropy analysis on each of the delta wave band signal B1, the theta wave band signal B2, the alpha wave band signal B3, the beta wave band signal B4, and the gamma wave band signal B5. The entropy analysis unit 131 obtains, through this entropy analysis, entropies of the delta wave band signal B1, the theta wave band signal B2, the alpha wave band signal B3, the beta wave band signal B4, and the gamma wave band signal B5 (a delta wave band signal entropy DB1, a theta wave band signal entropy DB2, an alpha wave band signal entropy DB3, a beta wave band signal entropy DB4, and a gamma wave band signal entropy DB5), separately.

The entropy analysis unit 131 performs entropy analysis on each of the delta wave band signal amplitude Ca1, the theta wave band signal amplitude Ca2, the alpha wave band signal amplitude Ca3, the beta wave band signal amplitude Ca4, and the gamma wave band signal amplitude Ca5. The entropy analysis unit 131 obtains, through this entropy analysis, entropies of the delta wave band signal amplitude Ca1, the theta wave band signal amplitude Ca2, the alpha wave band signal amplitude Ca3, the beta wave band signal amplitude Ca4, and the gamma wave band signal amplitude Ca5 (a delta wave band signal amplitude modulation entropy Da1, a theta wave band signal amplitude modulation entropy Da2, an alpha wave band signal amplitude modulation entropy Da3, a beta wave band signal amplitude modulation entropy Da4, and a gamma wave band signal amplitude modulation entropy Da5), separately.

The entropy analysis unit 131 performs entropy analysis on each of the delta wave band signal phase Cp1, the theta wave band signal phase Cp2, the alpha wave band signal phase Cp3, the beta wave band signal phase Cp4, and the gamma wave band signal phase Cp5. The entropy analysis unit 131 obtains, through this entropy analysis, entropies of the delta wave band signal phase Cp1, the theta wave band signal phase Cp2, the alpha wave band signal phase Cp3, the beta wave band signal phase Cp4, and the gamma wave band signal phase Cp5 (a delta wave band signal phase modulation entropy Dp1, a theta wave band signal phase modulation entropy Dp2, an alpha wave band signal phase modulation entropy Dp3, a beta wave band signal phase modulation entropy Dp4, and a gamma wave band signal phase modulation entropy Dp5), separately.

Note that when performing entropy analysis on the phases (the delta wave band signal phase Cp1, the theta wave band signal phase Cp2, the alpha wave band signal phase Cp3, the beta wave band signal phase Cp4, and the gamma wave band signal phase Cp5), the entropy analysis unit 131 performs entropy analysis on cosines "cosθ(t)" of the phases "θ(t)". This is because when the phase "θ(t)" is used as it is, a discontinuous point occurs every 2π [radian]. By the cosine "cosθ(t)" of the phase "θ(t)", the phase "θ(t)" can be treated as a continuous value. Therefore, the entropy analysis for the phase "θ(t)" calculates the entropy of the cosine "cosθ(t)" of the phase "θ(t)" by performing the entropy analysis for the cosine "cosθ(t)" of the phase "θ(t)".

Note that the entropy analysis for the phase "θ(t)" is not limited to calculating the entropy of the cosine "cosθ(t)" of the phase "θ(t)" by performing the entropy analysis on the cosine "cosθ(t)" of the phase "θ(t)", and the entropy analysis for the phase "θ(t)" may be performed by another method.

The fractal analysis unit 132 performs fractal analysis on the training electroencephalogram data A. Through this fractal analysis, the fractal analysis unit 132 obtains a fractal of the training electroencephalogram data A (an electroencephalogram fractal EA).

The fractal analysis unit 132 performs fractal analysis on each of the delta wave band signal amplitude Ca1, the theta wave band signal amplitude Ca2, the alpha wave band signal amplitude Ca3, the beta wave band signal amplitude Ca4, and the gamma wave band signal amplitude Ca5. The fractal analysis unit 132 obtains, through this fractal analysis, fractals of the delta wave band signal amplitude Ca1, the theta wave band signal amplitude Ca2, the alpha wave band signal amplitude Ca3, the beta wave band signal amplitude Ca4, and the gamma wave band signal amplitude Ca5 (a delta wave band signal amplitude fractal Ea1, a theta wave band signal amplitude fractal Ea2, an alpha wave band signal amplitude fractal Ea3, a beta wave band signal amplitude fractal Ea4, and a gamma wave band signal amplitude fractal Ea5), separately.

Results of the complexity analysis processing by the electroencephalogram processing unit 100 (the electroencephalogram entropy DA; the delta wave band signal entropy DB1, the theta wave band signal entropy DB2, the alpha wave band signal entropy DB3, the beta wave band signal entropy DB4, and the gamma wave band signal entropy DB5; the delta wave band signal amplitude modulation entropy Da1, the theta wave band signal amplitude modulation entropy Da2, the alpha wave band signal amplitude modulation entropy Da3, the beta wave band signal amplitude modulation entropy Da4, and the gamma wave band signal amplitude modulation entropy Da5; the delta wave band signal phase modulation entropy Dp1, the theta wave band signal phase modulation entropy Dp2, the alpha wave band signal phase modulation entropy Dp3, the beta wave band signal phase modulation entropy Dp4, and the gamma wave band signal phase modulation entropy Dp5; the electroencephalogram fractal EA; and the delta wave band signal amplitude fractal Ea1, the theta wave band signal amplitude fractal Ea2, the alpha wave band signal amplitude fractal Ea3, the beta wave band signal amplitude fractal Ea4, and the gamma wave band signal amplitude fractal Ea5) are transmitted to the machine learning unit 200.

The machine learning unit 200 performs machine learning processing of the machine learning model 210 for determining sleep/wakefulness stages (wakefulness, non-REM sleep, REM sleep) using the results of the complexity analysis processing received from the electroencephalogram processing unit 100 and label data Fa.

Note that in the machine learning processing of the machine learning model 210, the machine learning unit 200 may use all of the results of the complexity analysis processing received from the electroencephalogram processing unit 100, or may select and use some of the results of the complexity analysis processing received from the electroencephalogram processing unit 100.

The label data Fa is generated in advance for each epoch of the training electroencephalogram data A. The label data Fa has ground truth labels, and each ground truth label indicates one of the sleep/wakefulness stages, namely "wakefulness", "non-REM sleep", or "REM sleep" corresponding to each epoch of the training electroencephalogram data A.

For example, the machine learning unit 200 divides the results of the complexity analysis processing into three types; training data, verification data, and test data. The machine learning unit 200 then confirms accuracy of verification of the machine learning model 210, which is trained by training and validation using the training data, the verification data, and corresponding label data Fa, using the test data and corresponding label data Fa.

The machine learning model 210 trained by the machine learning unit 200 is used as the determination model 210a in the sleep/wakefulness stages determination device 2a.

### Sleep/Wakefulness Stages Determination Device

The sleep/wakefulness stages determination device 2a according to the first embodiment will be described with reference to FIG. 2. The sleep/wakefulness stages determination device 2a receives electroencephalogram data G. The electroencephalogram data G is electroencephalogram time-series data measured from one mouse (living body), and is a subject for determining the sleep/wakefulness stages.

The sleep/wakefulness stages determination device 2a includes an electroencephalogram processing unit 100 and a determination unit 300. The electroencephalogram processing unit 100 is the same as the electroencephalogram processing unit 100 of the information processing device 1a illustrated in FIG. 1, and therefore, a description thereof is omitted.

In the sleep/wakefulness stages determination device 2a, the electroencephalogram processing unit 100 transmits results of complexity analysis processing obtained from the electroencephalogram data G (an electroencephalogram entropy DA; a delta wave band signal entropy DB1, a theta wave band signal entropy DB2, an alpha wave band signal entropy DB3, a beta wave band signal entropy DB4, and a gamma wave band signal entropy DB5; a delta wave band signal amplitude modulation entropy Da1, a theta wave band signal amplitude modulation entropy Da2, an alpha wave band signal amplitude modulation entropy Da3, a beta wave band signal amplitude modulation entropy Da4, and a gamma wave band signal amplitude modulation entropy Da5; a delta wave band signal phase modulation entropy Dp1, a theta wave band signal phase modulation entropy Dp2, an alpha wave band signal phase modulation entropy Dp3, a beta wave band signal phase modulation entropy Dp4, and a gamma wave band signal phase modulation entropy Dp5; an electroencephalogram fractal EA; and a delta wave band signal amplitude fractal Ea1, a theta wave band signal amplitude fractal Ea2, an alpha wave band signal amplitude fractal Ea3, a beta wave band signal amplitude fractal Ea4, and a gamma wave band signal amplitude fractal Ea5) to the determination unit 300.

The determination unit 300 determines the sleep/wakefulness stages (wakefulness, non-REM sleep, REM sleep) using the determination model 210a, from the results of the complexity analysis processing DA, DB1 to DB5, EA, Da1 to Da5, Dp1 to Dp5, and Ea1 to Ea5 received from the electroencephalogram processing unit 100. The determination unit 300 outputs a sleep/wakefulness stages determination result H. The sleep/wakefulness stages determination result H may be information that specifies one of "wakefulness", "non-REM sleep", or "REM sleep", or may be confidence of each of "wakefulness", "non-REM sleep", and "REM sleep".

According to the present embodiment, the machine learning processing of the machine learning model for determining the sleep/wakefulness stages (wakefulness, non-REM sleep, REM sleep) is performed using the results of the complexity analysis processing on both of the amplitude and the phase for each of the predetermined plurality of frequency bands included in the electroencephalogram of a living body that is a mammal, and the label data of the sleep/wakefulness stages corresponding to the results. Here, in the electroencephalogram, each frequency band, the amplitude of each frequency band, and the phase of each frequency band each have different physiological significance. In general, the amplitudes of the delta wave band and the theta wave band are referenced for the determination of the sleep/wakefulness stages, but differences between the stages have also been reported in the high frequency bands. Therefore, by performing the complexity analysis processing on both of the amplitude and the phase for each of the predetermined plurality of frequency bands included in the electroencephalogram, and performing the machine learning processing of the machine learning model for determining the sleep/wakefulness stages (wakefulness, non-REM sleep, REM sleep) using the results of the complexity analysis processing and the label data of the sleep/wakefulness stages corresponding to the results, the performance of the determination model, which is a result of the machine learning of the machine learning model, is improved. This provides an effect of contributing to improvement of the accuracy of automated determination of the sleep/wakefulness stages.

In the example described above, the electroencephalogram processing unit 100 performs, as the complexity analysis processing,
the entropy analysis on:
   - the training electroencephalogram data A;
   - the delta wave band signal B1, the theta wave band signal B2, the alpha wave band signal B3, the beta wave band signal B4, and the gamma wave band signal B5;
   - the delta wave band signal amplitude Ca1, the theta wave band signal amplitude Ca2, the alpha wave band signal amplitude Ca3, the beta wave band signal amplitude Ca4, and the gamma wave band signal amplitude Ca5; and
   - the delta wave band signal phase Cp1, the theta wave band signal phase Cp2, the alpha wave band signal phase Cp3, the beta wave band signal phase Cp4, and the gamma wave band signal phase Cp5, and
the fractal analysis on:
   - the training electroencephalogram data A; and
   - the delta wave band signal amplitude Ca1, the theta wave band signal amplitude Ca2, the alpha wave band signal amplitude Ca3, the beta wave band signal amplitude Ca4, and the gamma wave band signal amplitude Ca5. The machine learning unit 200 uses the results of this complexity analysis processing for machine learning.

As another example, the electroencephalogram processing unit 100 may perform, as the complexity analysis processing,
the entropy analysis on:
   - the delta wave band signal amplitude Ca1, the theta wave band signal amplitude Ca2, the alpha wave band signal amplitude Ca3, the beta wave band signal amplitude Ca4, and the gamma wave band signal amplitude Ca5; and
   - the delta wave band signal phase Cp1, the theta wave band signal phase Cp2, the alpha wave band signal phase Cp3, the beta wave band signal phase Cp4, and the gamma wave band signal phase Cp5, and
the fractal analysis on:
   - the delta wave band signal amplitude Ca1, the theta wave band signal amplitude Ca2, the alpha wave band signal amplitude Ca3, the beta wave band signal amplitude Ca4, and the gamma wave band signal amplitude Ca5. The machine learning unit 200 uses the results of this complexity analysis processing for machine learning.

As another example, the electroencephalogram processing unit 100 may perform, as the complexity analysis processing,
the entropy analysis on:
   - the delta wave band signal B1, the theta wave band signal B2, the alpha wave band signal B3, the beta wave band signal B4, and the gamma wave band signal B5;
   - the delta wave band signal amplitude Ca1, the theta wave band signal amplitude Ca2, the alpha wave band signal amplitude Ca3, the beta wave band signal amplitude Ca4, and the gamma wave band signal amplitude Ca5; and
   - the delta wave band signal phase Cp1, the theta wave band signal phase Cp2, the alpha wave band signal phase Cp3, the beta wave band signal phase Cp4, and the gamma wave band signal phase Cp5, and
the fractal analysis on:
   - the delta wave band signal amplitude Ca1, the theta wave band signal amplitude Ca2, the alpha wave band signal amplitude Ca3, the beta wave band signal amplitude Ca4, and the gamma wave band signal amplitude Ca5. The machine learning unit 200 uses the results of this complexity analysis processing for machine learning.

Note that the electroencephalogram processing unit 100 may perform, as the complexity analysis processing, at least the entropy analysis, or both of the entropy analysis and the fractal analysis. Therefore, the electroencephalogram processing unit 100 does not necessarily need to perform the fractal analysis as the complexity analysis processing. When the fractal analysis is not performed as the complexity analysis processing, only the results of the entropy analysis are transmitted to the machine learning unit 200 as the results of the complexity analysis processing and used for machine learning.

### Second Embodiment

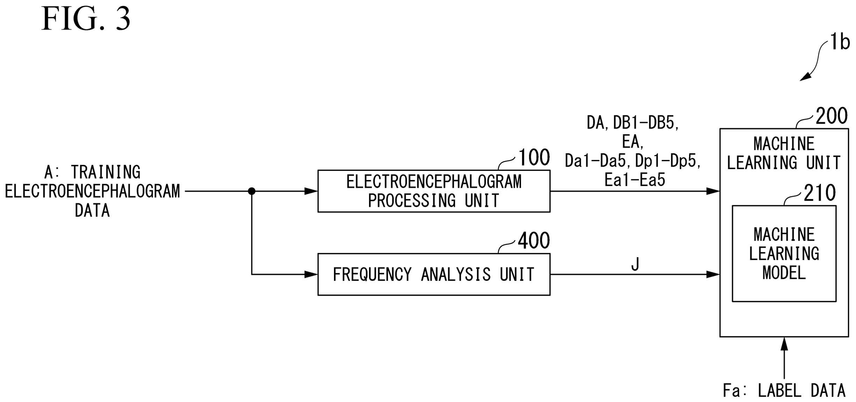

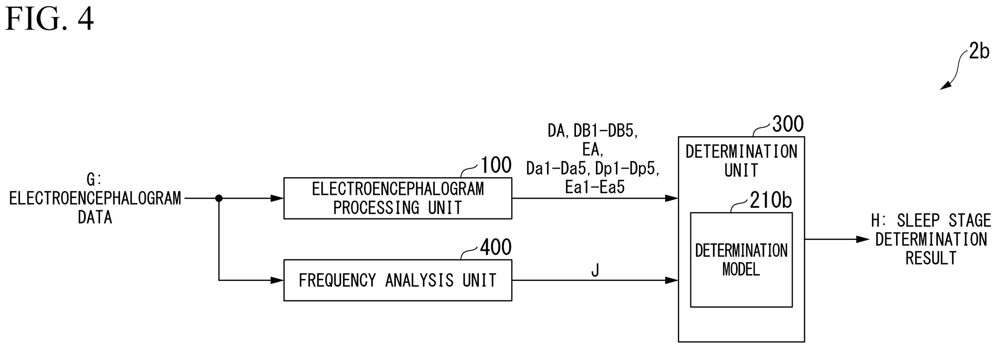

A second embodiment will be described with reference to FIGS. 3 and 4. FIG. 3 is a block diagram illustrating a configuration example of an information processing device according to the second embodiment. FIG. 4 is a block diagram illustrating a configuration example of a sleep/wakefulness stages determination device according to the second embodiment. In FIGS. 3 and 4, the same reference numerals are given to parts corresponding to the parts in FIGS. 1 and 2, and descriptions thereof will be omitted.

### Information Processing Device

In FIG. 3, an information processing device 1b further includes a frequency analysis unit 400 compared to the information processing device 1a in FIG. 1. The frequency analysis unit 400 converts training electroencephalogram data A for each epoch into a frequency spectrum J. The epochs are common to an electroencephalogram processing unit 100 and the frequency analysis unit 400. The frequency analysis unit 400 transmits the frequency spectrum J to a machine learning unit 200.

The machine learning unit 200 performs machine learning processing of a machine learning model 210 for determining sleep/wakefulness stages (wakefulness, non-REM sleep, REM sleep) using results of complexity analysis processing DA, DB1 to DB5, EA, Da1 to Da5, Dp1 to Dp5, and Ea1 to Ea5 received from the electroencephalogram processing unit 100, the frequency spectrum J received from the frequency analysis unit 400, and label data Fa.

Note that in the machine learning processing of the machine learning model 210, the machine learning unit 200 may use all of the results of the complexity analysis processing received from the electroencephalogram processing unit 100 and the frequency spectrum received from the frequency analysis unit 400, or may select and use some of the results of the complexity analysis processing received from the electroencephalogram processing unit 100 and the frequency spectrum received from the frequency analysis unit 400.

The label data Fa is generated in advance for each epoch of the training electroencephalogram data A. The label data Fa has ground truth labels, and each ground truth label indicates one of the sleep/wakefulness stages, namely "wakefulness", "non-REM sleep", or "REM sleep" corresponding to each epoch of the training electroencephalogram data A.

The machine learning model 210 trained by the machine learning unit 200 is used as a determination model 210b in a sleep/wakefulness stages determination device 2b.

### Sleep/Wakefulness Stages Determination Device

In FIG. 4, the sleep/wakefulness stages determination device 2b further includes a frequency analysis unit 400 compared to the sleep/wakefulness stages determination device 2a in FIG. 2. The frequency analysis unit 400 converts electroencephalogram data G for each epoch into a frequency spectrum J. The epochs are common to an electroencephalogram processing unit 100 and the frequency analysis unit 400. The frequency analysis unit 400 transmits the frequency spectrum J to a determination unit 300.

The determination unit 300 determines sleep/wakefulness stages (wakefulness, non-REM sleep, REM sleep) using the determination model 210b, from results of complexity analysis processing DA, DB1 to DB5, EA, Da1 to Da5, Dp1 to Dp5, and Ea1 to Ea5 received from the electroencephalogram processing unit 100, and the frequency spectrum J received from the frequency analysis unit 400. The determination unit 300 outputs a sleep/wakefulness stages determination result H. The sleep/wakefulness stages determination result H may be information that specifies one of "wakefulness", "non-REM sleep", or "REM sleep", or may be confidence of each of "wakefulness", "non-REM sleep", and "REM sleep".

According to the present embodiment, compared to the first embodiment described above, the machine learning processing of the machine learning model is performed further using the frequency spectrum of the electroencephalogram. This provides an effect of further improving the performance of the determination model that is a result of machine learning of the machine learning model.

### Third Embodiment

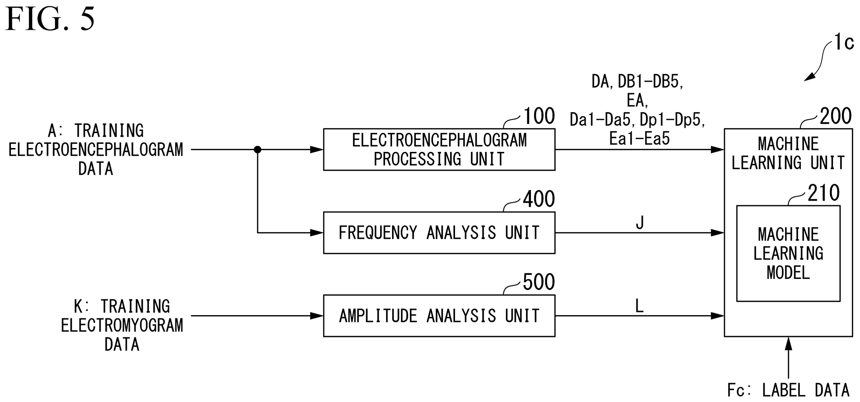

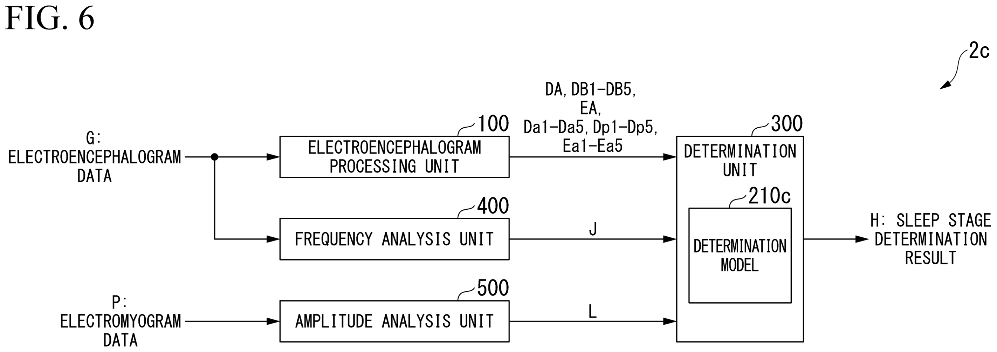

A third embodiment will be described with reference to FIGS. 5 and 6. FIG. 5 is a block diagram illustrating a configuration example of an information processing device according to the third embodiment. FIG. 6 is a block diagram illustrating a configuration example of a sleep/wakefulness stages determination device according to the third embodiment. In FIGS. 5 and 6, the same reference numerals are given to parts corresponding to the parts in FIGS. 1 to 4, and descriptions thereof will be omitted.

### Information Processing Device

In FIG. 5, an information processing device 1c further includes an amplitude analysis unit 500 compared to the information processing device 1b in FIG. 3. The amplitude analysis unit 500 obtains an amplitude L for each epoch of training electromyogram data K. The training electromyogram data K is electromyogram time-series data measured from the same mouse (living body) from which training electroencephalogram data A is measured, and is used for machine learning of a machine learning model 210. The epochs of the amplitude analysis unit 500 are common to epochs of an electroencephalogram processing unit 100 and a frequency analysis unit 400. The amplitude analysis unit 500 transmits the amplitude L to a machine learning unit 200.

The machine learning unit 200 performs machine learning processing of the machine learning model 210 for determining sleep/wakefulness stages (wakefulness, non-REM sleep, REM sleep) using results of complexity analysis processing DA, DB1 to DB5, EA, Da1 to Da5, Dp1 to Dp5, and Ea1 to Ea5 received from the electroencephalogram processing unit 100, a frequency spectrum J received from the frequency analysis unit 400, the amplitude L received from the amplitude analysis unit 500, and label data Fc.

Note that in the machine learning processing of the machine learning model 210, the machine learning unit 200 may use all of the results of the complexity analysis processing received from the electroencephalogram processing unit 100, the frequency spectrum received from the frequency analysis unit 400, and the amplitude received from the amplitude analysis unit 500 or may select and use some of the results of the complexity analysis processing received from the electroencephalogram processing unit 100, the frequency spectrum received from the frequency analysis unit 400, and the amplitude received from the amplitude analysis unit 500.

The label data Fc, which is a label data common to both of the training electroencephalogram data A and the training electromyogram data K, is generated in advance for each epoch that is common to both of the training electroencephalogram data A and the training electromyogram data K. The label data Fc has ground truth labels, and each ground truth label indicates one of sleep/wakefulness stages, namely "wakefulness", "non-REM sleep", or "REM sleep" corresponding to each epoch that is common to both of the training electroencephalogram data A and the training electromyogram data K.

The machine learning model 210 trained by the machine learning unit 200 is used as a determination model 210c in a sleep/wakefulness stages determination device 2c.

### Sleep/Wakefulness Stages Determination Device

In FIG. 6, the sleep/wakefulness stages determination device 2c further includes an amplitude analysis unit 500 compared to the sleep/wakefulness stages determination device 2b in FIG. 4. The amplitude analysis unit 500 obtains an amplitude L for each epoch of electromyogram data P. The electromyogram data P is electromyogram time-series data measured from the same mouse (living body) from which electroencephalogram data G is measured, and is a subject for determining the sleep/wakefulness stages. The epoch of the amplitude analysis unit 500 is common to an electroencephalogram processing unit 100 and a frequency analysis unit 400. The amplitude analysis unit 500 transmits the amplitude L to a determination unit 300.

The determination unit 300 determines sleep/wakefulness stages (wakefulness, non-REM sleep, REM sleep) using the determination model 210c, from results of complexity analysis processing DA, DB1 to DB5, EA, Da1 to Da5, Dp1 to Dp5, and Ea1 to Ea5 received from the electroencephalogram processing unit 100, a frequency spectrum J received from the frequency analysis unit 400, and the amplitude L received from the amplitude analysis unit 500. The determination unit 300 outputs a sleep/wakefulness stages determination result H. The sleep/wakefulness stages determination result H may be information that specifies one of "wakefulness", "non-REM sleep", or "REM sleep", or may be confidence of each of "wakefulness", "non-REM sleep", and "REM sleep".

According to the present embodiment, compared to the second embodiment described above, the machine learning processing of the machine learning model is performed further using the amplitude of the electromyogram. This provides an effect of further improving the performance of the determination model that is a result of machine learning of the machine learning model.

### Fourth Embodiment

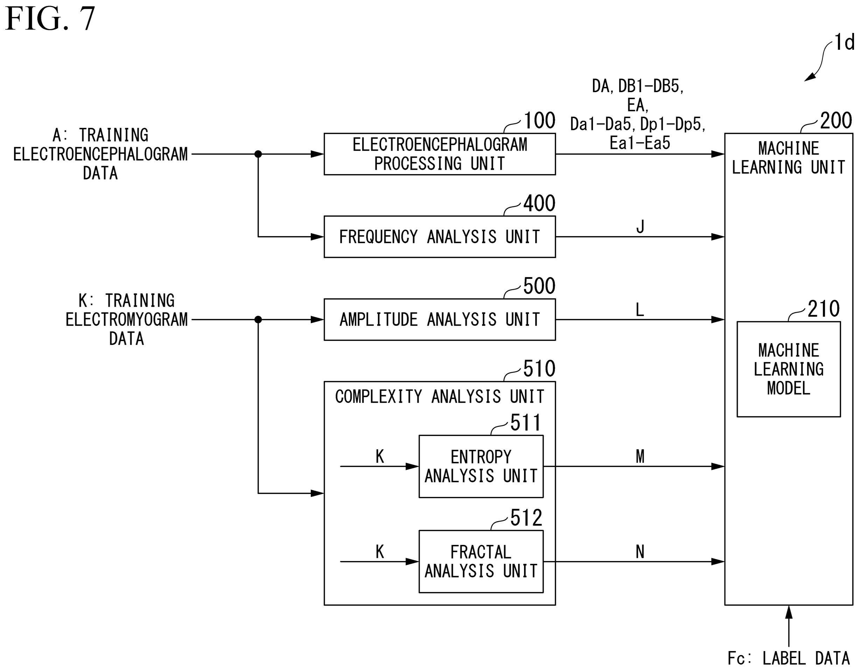

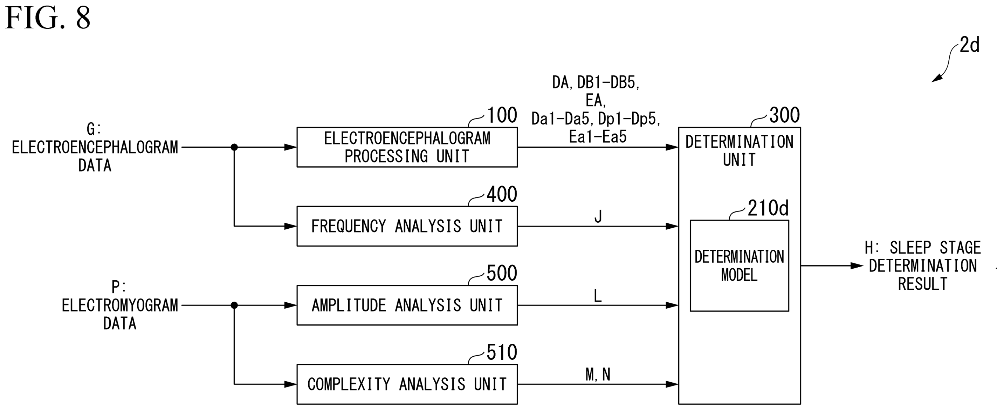

A fourth embodiment will be described with reference to FIGS. 7 and 8. FIG. 7 is a block diagram illustrating a configuration example of an information processing device according to the fourth embodiment. FIG. 8 is a block diagram illustrating a configuration example of a sleep/wakefulness stages determination device according to the fourth embodiment. In FIGS. 7 and 8, the same reference numerals are given to parts corresponding to the parts in FIGS. 1 to 6, and descriptions thereof will be omitted.

### Information Processing Device

In FIG. 7, an information processing device 1d further includes a complexity analysis unit 510 compared to the information processing device 1c in FIG. 5. The complexity analysis unit 510 comprises an entropy analysis unit 511 and a fractal analysis unit 512. The complexity analysis unit 510 performs complexity analysis processing for each epoch of training electromyogram data K. The complexity analysis processing comprises entropy analysis by the entropy analysis unit 511 and fractal analysis by the fractal analysis unit 512. The epochs of the complexity analysis unit 510 are common to epochs of an electroencephalogram processing unit 100, a frequency analysis unit 400, and an amplitude analysis unit 500.

The entropy analysis unit 511 performs entropy analysis on the training electromyogram data K. The entropy analysis unit 511 obtains an entropy of the training electromyogram data K (an electromyogram entropy M) through this entropy analysis.

The fractal analysis unit 512 performs fractal analysis on the training electromyogram data K. The fractal analysis unit 512 obtains a fractal of the training electromyogram data K (an electromyogram fractal N) through this fractal analysis.

The complexity analysis unit 510 transmits results of the complexity analysis processing (the electromyogram entropy M and the electromyogram fractal N) to a machine learning unit 200.

The machine learning unit 200 performs machine learning processing of a machine learning model 210 for determining sleep/wakefulness stages (wakefulness, non-REM sleep, REM sleep) using results DA, DB1 to DB5, EA, Da1 to Da5, Dp1 to Dp5, and Ea1 to Ea5 received from the electroencephalogram processing unit 100, a frequency spectrum J received from the frequency analysis unit 400, an amplitude L received from the amplitude analysis unit 500, the complexity analysis processing results M and N received from the complexity analysis unit 510, and label data Fc.

Note that in the machine learning processing of the machine learning model 210, the machine learning unit 200 may use all of the results of the complexity analysis processing received from the electroencephalogram processing unit 100, the frequency spectrum received from the frequency analysis unit 400, the amplitude received from the amplitude analysis unit 500, and the results of the complexity analysis processing received from the complexity analysis unit 510, or may select and use some of the results of the complexity analysis processing received from the electroencephalogram processing unit 100, the frequency spectrum received from the frequency analysis unit 400, the amplitude received from the amplitude analysis unit 500, and the results of the complexity analysis processing received from the complexity analysis unit 510.

The label data Fc, which is a label data common to both of the training electroencephalogram data A and the training electromyogram data K, is generated in advance for each epoch that is common to both of the training electroencephalogram data A and the training electromyogram data K. The label data Fc has ground truth labels, and each ground truth label indicates one of sleep/wakefulness stages, namely "wakefulness", "non-REM sleep", or "REM sleep" corresponding to each epoch that is common to both of the training electroencephalogram data A and the training electromyogram data K.

The machine learning model 210 trained by the machine learning unit 200 is used as a determination model 210d in a sleep/wakefulness stages determination device 2d.

### Sleep/Wakefulness Stages Determination Device

In FIG. 8, the sleep/wakefulness stages determination device 2d further includes a complexity analysis unit 510 compared to the sleep/wakefulness stages determination device 2c in FIG. 6. The complexity analysis unit 510 obtains an electromyogram entropy M and an electromyogram fractal N for each epoch of electromyogram data P. The epochs of the complexity analysis unit 510 are common to epochs of an electroencephalogram processing unit 100, a frequency analysis unit 400, and an amplitude analysis unit 500. The complexity analysis unit 510 transmits results of the complexity analysis processing (the electromyogram entropy M and the electromyogram fractal N) to a determination unit 300.

The determination unit 300 determines sleep/wakefulness stages (wakefulness, non-REM sleep, REM sleep) using the determination model 210d, from results of complexity analysis processing DA, DB1 to DB5, EA, Da1 to Da5, Dp1 to Dp5, and Ea1 to Ea5 received from the electroencephalogram processing unit 100, a frequency spectrum J received from the frequency analysis unit 400, an amplitude L received from the amplitude analysis unit 500, and the complexity analysis processing results M and N received from the complexity analysis unit 510. The determination unit 300 outputs a sleep/wakefulness stages determination result H. The sleep/wakefulness stages determination result H may be information that specifies one of "wakefulness", "non-REM sleep", or "REM sleep", or may be confidence of each of "wakefulness", "non-REM sleep", and "REM sleep".

According to the present embodiment, compared to the third embodiment described above, the machine learning processing of the machine learning model is performed further using the results of the complexity analysis processing of the electromyogram. This provides an effect of further improving the performance of the determination model that is a result of machine learning of the machine learning model. According to the fourth embodiment, experimental verification showed that accuracy of automated determination of sleep/wakefulness stages in mice, which has been about 96% in the past, was improved to 98% or more.

In the embodiments described above, a mouse is used as an example of a mammal, but the embodiments are applicable to other mammals such as a human and a monkey other than the mouse.

Note that while human sleep analysis typically involves polysomnography and therefore includes wide variety of data types in addition to electroencephalograms and electromyograms, in mice, available data is often limited to only electroencephalograms and electromyograms. Therefore, in mice, using the results of the complexity analysis on the electroencephalogram or the electromyogram according to the embodiments described above is particularly effective.

Further, the functions of the information processing devices 1a to 1d are each implemented by the information processing devices 1a to 1d including computer hardware such as a central processing unit (CPU) and a memory, and the CPU executing a computer program stored in the memory. Note that the information processing devices 1a to 1d may be configured using general-purpose computer devices or may be configured as dedicated hardware devices. For example, the information processing devices 1a to 1d may be configured using server computers connected to a communication network such as the Internet. The functions of the information processing devices 1a to 1d each may be implemented through cloud computing. The information processing devices 1a to 1d may be implemented by individual computers, or the functions of the information processing devices 1a to 1d may be distributed and implemented across a plurality of computers. The information processing devices 1a to 1d may be configured to launch a website using, for example, a WWW system or the like.

The functions of the sleep/wakefulness stages determination devices 2a to 2d each are implemented by the sleep/wakefulness stages determination devices 2a to 2d including computer hardware such as a CPU and a memory, and the CPU executing a computer program stored in the memory. Note that the sleep/wakefulness stages determination devices 2a to 2d may be configured using general-purpose computer devices, or may be configured as dedicated hardware devices. For example, the sleep/wakefulness stages determination devices 2a to 2d may be configured using server computers connected to a communication network such as the Internet. The functions of the sleep/wakefulness stages determination devices 2a to 2d may each be implemented through cloud computing. The sleep/wakefulness stages determination devices 2a to 2d may be implemented by individual computers, or the functions of the sleep/wakefulness stages determination devices 2a to 2d may be distributed and implemented across a plurality of computers. The sleep/wakefulness stages determination device 2a to 2d may be configured to launch a website using, for example, a WWW system or the like.

The information processing device and the sleep/wakefulness stages determination device described above may be configured as separate devices or may be configured as a single information processing device.

In the embodiments described above, entropy analysis, fractal analysis, frequency analysis, and the like are used as analysis methods for electroencephalogram data, and amplitude analysis, entropy analysis, fractal analysis, and the like are used as analysis methods for electromyogram data. However, other analysis methods other than these may also be applied.

Although the embodiments of the present disclosure have been described in detail above with reference to the drawings, the specific configurations are not limited to these embodiments, and include design modifications and the like that do not depart from the scope of the present disclosure.

For example, in the embodiments described above, the sleep/wakefulness stages determination devices 2a to 2d use the determination models 210a to 210d generated by the information processing devices 1a to 1d using machine learning, but methods for generating the determination models 210a to 210d are not limited thereto.

For example, the sleep/wakefulness stages determination device 2a in FIG. 2 only needs to include the determination model 210a that determines the sleep/wakefulness stages (wakefulness, non-REM sleep, REM sleep) from the results of the complexity analysis processing DA, DB1 to DB5, EA, Da1 to Da5, Dp1 to Dp5, and Ea1 to Ea5 received from the electroencephalogram processing unit 100, and a method for generating the determination model 210a is not limited.

For example, the sleep/wakefulness stages determination device 2b in FIG. 4 only needs to include the determination model 210b that determines the sleep/wakefulness stages (wakefulness, non-REM sleep, REM sleep) from the results of the complexity analysis processing DA, DB1 to DB5, EA, Da1 to Da5, Dp1 to Dp5, and Ea1 to Ea5 received from the electroencephalogram processing unit 100 and the frequency spectrum J received from the frequency analysis unit 400, and a method for generating the determination model 210b is not limited.

For example, the sleep/wakefulness stages determination device 2c in FIG. 6 only needs to include the determination model 210c that determines the sleep/wakefulness stages (wakefulness, non-REM sleep, REM sleep) from the results of the complexity analysis processing DA, DB1 to DB5, EA, Da1 to Da5, Dp1 to Dp5, and Ea1 to Ea5 received from the electroencephalogram processing unit 100, the frequency spectrum J received from the frequency analysis unit 400, and the amplitude L received from the amplitude analysis unit 500, and a method for generating the determination model 210c is not limited.

For example, the sleep/wakefulness stages determination device 2d in FIG. 8 only needs to include the determination model 210d that determines the sleep/wakefulness stages (wakefulness, non-REM sleep, REM sleep) from the results of the complexity analysis processing DA, DB1 to DB5, EA, Da1 to Da5, Dp1 to Dp5, and Ea1 to Ea5 received from the electroencephalogram processing unit 100, the frequency spectrum J received from the frequency analysis unit 400, the amplitude L received from the amplitude analysis unit 500, and the results of the complexity analysis processing M and N received from the complexity analysis unit 510, and a method for generating the determination model 210d is not limited.

In the embodiments described above, the training electroencephalogram data A and the electroencephalogram data G may be data obtained by spline interpolation. According to the spline interpolation, electroencephalograms measured under various conditions can be applied regardless of a sampling period of the electroencephalogram device.

In the embodiments described above, a moving average may be used in the entropy analysis. To be specific, when calculating a value that indicates a degree of irregularity in a time series (entropy) for a subject on which entropy analysis is performed, an autocorrelation is calculated by shifting a window of a fixed width and using an average of the time series within the window as a target. By using the moving average in the entropy analysis, an effect of improving accuracy of analysis for short-term data is obtained.

In the embodiments described above, expanded sample entropy (expSampEn) may be used as the entropy analysis. The expanded sample entropy calculates an autocorrelation of a single point to all in a time series when determining a value that indicates a degree of irregularity in a time series (entropy) of a subject on which entropy analysis is performed. According to the expanded sample entropy, temporal resolution is improved, enabling a highly accurate entropy analysis.

Note that sample entropy (SampEn), approximate entropy (ApEn), or the like may be used for the entropy analysis. However, using the expanded sample entropy enables faster entropy analysis than the sample entropy and the approximate entropy.

In the embodiments described above, when generating learning data used for machine learning of the machine learning model 210, the machine learning unit 200 may expand the learning data by standardization or normalization using a plurality of thresholds. The machine learning unit 200 may use only the standardization, may use only the normalization using the plurality of thresholds, or may use both of the standardization and the normalization using the plurality of thresholds.

In the embodiments described above, since the entropy analysis is logarithmic nonlinear processing, it can be expected to have an effect of making machine learning convergence more efficiently and quickly.

In the embodiments described above, a rescoring short epoch (RSE) method may be applied to the determination result of the sleep/wakefulness stages (wakefulness, non-REM sleep, REM sleep) determined using the determination model (the sleep/wakefulness stages determination result H). In this case, the sleep/wakefulness stages determination device includes an RSE method execution unit that executes the RSE method. When a judge for the sleep/wakefulness stages visually inspects the sleep/wakefulness stages determination result H to make a final determination of the sleep/wakefulness stages, the visual determination of the sleep/wakefulness stages generally uses a determination rule that one stage continues for 8 seconds to 20 seconds. However, in ambiguous epochs such as stage turning points, predictions made by machine learning tend to become unstable, resulting in stages that last for a shorter duration than a certain period of time. The RSE method execution unit uses the RSE method as an algorithm for eliminating epochs having short stage durations by lowering priority of epochs determined to have stage durations shorter than a certain time, starting from an epoch having the shortest stage duration, in the determination results of the sleep/wakefulness stages. By applying the RSE method to the sleep/wakefulness stages determination results H, workload of visually determining the sleep/wakefulness stages by the judge can be significantly reduced. This provides an effect of improving accuracy of the final determination of sleep/wakefulness stages by the judge for the sleep/wakefulness stages. In the experiment, the accuracy of the final determination of sleep/wakefulness stages improved by approximately 0.1% to 1.0% when the RSE method was applied compared to when the RSE method was not applied.

Note that, while learning temporal changes, as in a known hidden Markov model (HMM) or recurrent neural network (RNN), reduces the accuracy of determining the sleep/wakefulness stages in disease model mice, applying the RSE method maintains applicability to sleep architecture abnormalities by correcting the sleep/wakefulness stages determination results without learning temporal changes.

In the embodiments described above, it may be possible to suppress display of epochs with a sufficiently high degree of confidence among the determination results of the sleep/wakefulness stages (wakefulness, non-REM sleep, REM sleep) determined using the determination model (the sleep/wakefulness stages determination results H). In this case, the sleep/wakefulness stages determination device includes a skipping unit that suppresses the display of those epochs. This significantly reduces the workload of the judge for the sleep/wakefulness stages to visually determine the sleep/wakefulness stages.

The accuracy of the determination may be displayed for the determination results (the sleep/wakefulness stages determination results H) for the sleep/wakefulness stages (wakefulness, non-REM sleep, REM sleep) determined using the determination model.

In this case, the sleep/wakefulness stages determination device includes an accuracy display control unit that displays this accuracy. This enables the judge for the sleep/wakefulness stages to be provided with a general criteria for visual determination of the sleep/wakefulness stages, thereby contributing to improved efficiency in the visual determination of the sleep/wakefulness stages.

In the embodiments described above, deep learning may be applied as the machine learning. Therefore, in the embodiments, the machine learning may be deep learning or other types of machine learning besides deep learning.

### Fifth Embodiment

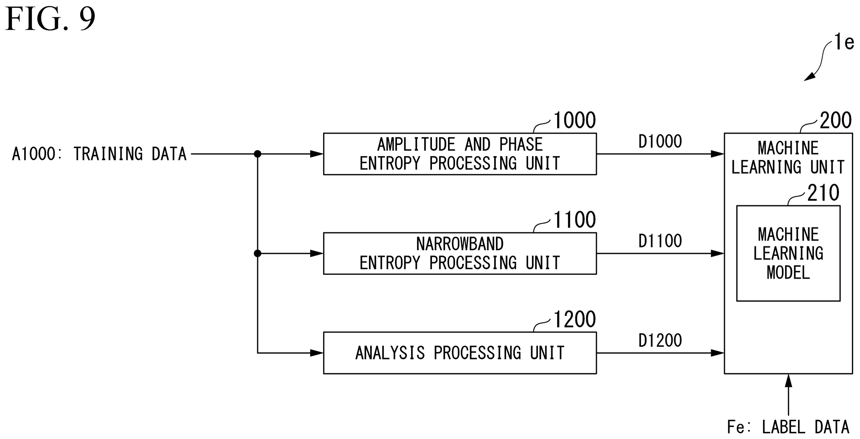

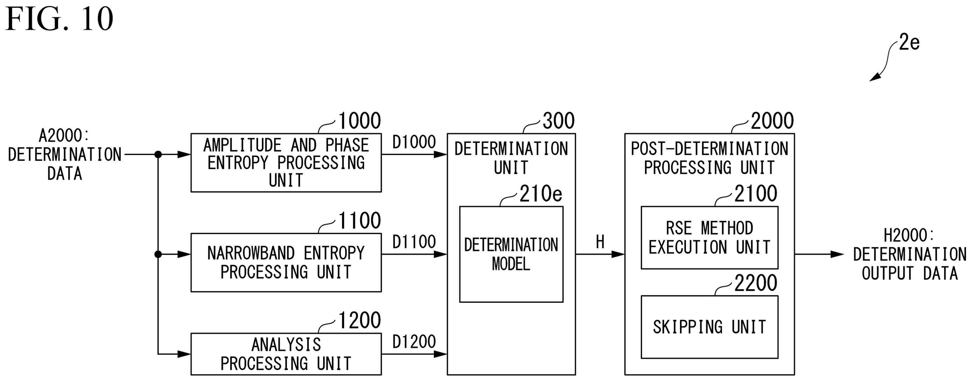

A fifth embodiment will be described with reference to FIGS. 9 and 10. FIG. 9 is a block diagram illustrating a configuration example of an information processing device according to the fifth embodiment. FIG. 10 is a block diagram illustrating a configuration example of a sleep/wakefulness stages determination device according to the fifth embodiment.

### Information Processing Device

An information processing device 1e according to the fifth embodiment will be described with reference to FIG. 9. Training data A1000 is input to the information processing device 1e. The training data A1000 may include only training electroencephalogram data A, only training electromyogram data K, or both of the training electroencephalogram data A and the training electromyogram data K. The type of data used as the training data A1000 is determined in advance. Note that the training electroencephalogram data A, the training electromyogram data K, or both of the training electroencephalogram data A and the training electromyogram data K may be data obtained by spline interpolation.

The information processing device 1e includes an amplitude and phase entropy processing unit 1000, a narrowband entropy processing unit 1100, an analysis processing unit 1200, and a machine learning unit 200.

The amplitude and phase entropy processing unit 1000 obtains an amplitude and a phase from the training data A1000 for each of a predetermined plurality of frequency bands, and performs entropy analysis on the obtained amplitude and phase in each frequency band. Examples of the predetermined plurality of frequency bands include a delta wave band, a theta wave band, an alpha wave band, a beta wave band, and a gamma wave band.

When the training data A1000 includes only the training electroencephalogram data A, the amplitude and phase entropy processing unit 1000 obtains the amplitude and the phase from the training electroencephalogram data A for each of the predetermined plurality of frequency bands, and performs entropy analysis on the obtained amplitude and phase in each frequency band.

When the training data A1000 includes only the training electromyogram data K, the amplitude and phase entropy processing unit 1000 obtains the amplitude and the phase from the training electromyogram data K for each of the predetermined plurality of frequency bands, and performs entropy analysis on the obtained amplitude and phase in each frequency band.

When the training data A1000 includes both of the training electroencephalogram data A and the training electromyogram data K, the amplitude and phase entropy processing unit 1000 obtains the amplitude and the phase from the training electroencephalogram data A for each of the predetermined plurality of frequency bands, and performs entropy analysis on the obtained amplitude and phase in each frequency band. Further, the amplitude and phase entropy processing unit 1000 obtains the amplitude and the phase from the training electromyogram data K for each of the predetermined plurality of frequency bands, and performs entropy analysis on the obtained amplitude and phase in each frequency band.

An entropy D1000, which is a result of the entropy analysis by the amplitude and phase entropy processing unit 1000, is transmitted to the machine learning unit 200.

Note that the amplitude and phase entropy processing unit 1000 may perform entropy analysis on a cosine of the phase when performing entropy analysis on the phase. Therefore, in the entropy analysis on the phase, the entropy of the cosine of the phase is calculated by performing entropy analysis on the cosine of the phase.

The narrowband entropy processing unit 1100 extracts signals in the predetermined plurality of frequency bands, separately, from the training data A1000, and performs entropy analysis on each of the extracted signals in each of the plurality of frequency bands. Examples of the predetermined plurality of frequency bands include a delta wave band, a theta wave band, an alpha wave band, a beta wave band, and a gamma wave band.

When the training data A1000 includes only the training electroencephalogram data A, the narrowband entropy processing unit 1100 extracts signals in the predetermined plurality of frequency bands, separately, from the training electroencephalogram data A, and performs entropy analysis on each of the extracted signals in each of the plurality of frequency bands.

When the training data A1000 includes only the training electromyogram data K, the narrowband entropy processing unit 1100 extracts signals in the predetermined plurality of frequency bands, separately, from the training electromyogram data K, and performs entropy analysis on each of the extracted signals in each of the plurality of frequency bands.

When the training data A1000 includes both of the training electroencephalogram data A and the training electromyogram data K, the narrowband entropy processing unit 1100 extracts signals in the predetermined plurality of frequency bands, separately, from the training electroencephalogram data A, and performs entropy analysis on each of the extracted signals in each of the plurality of frequency bands. Further, the narrowband entropy processing unit 1100 extracts signals in the predetermined plurality of frequency bands, separately, from the training electromyogram data K, and performs entropy analysis on each of the extracted signals in each of the plurality of frequency bands.

An entropy D1100, which is a result of the entropy analysis by the narrowband entropy processing unit 1100, is transmitted to the machine learning unit 200.

Note that the amplitude and phase entropy processing unit 1000 and the narrowband entropy processing unit 1100 may perform entropy analysis on data obtained by applying a moving average to the input data. The amplitude and phase entropy processing unit 1000 and the narrowband entropy processing unit 1100 may use, for example, expanded sample entropy, sample entropy, or approximate entropy as the entropy analysis. However, using the expanded sample entropy enables faster entropy analysis than the sample entropy and the approximate entropy.

The analysis processing unit 1200 performs predetermined analysis processing on the training data A1000. The analysis processing performed by the analysis processing unit 1200 may be, for example, one or more of frequency analysis of the electroencephalogram, amplitude analysis of the electromyogram, fractal analysis, and entropy analysis of broadband. In the entropy analysis of broadband, the entropy analysis is performed on the training data A1000 without extracting signals in the predetermined plurality of frequency bands from the training data A1000 as in the amplitude and phase entropy processing unit 1000 and the narrowband entropy processing unit 1100.

Analysis data D1200, which is a result of the analysis by the analysis processing unit 1200, is transmitted to the machine learning unit 200.

The machine learning unit 200 performs machine learning processing of the machine learning model 210 for determining sleep/wakefulness stages (wakefulness, non-REM sleep, REM sleep) using the entropy D1000, the entropy D1100, the analysis data D1200, and label data Fe.

The label data Fe is generated in advance for each epoch of the training data A1000.

When the training data A1000 includes only the training electroencephalogram data A, the label data Fe has a ground truth label indicating one of the sleep/wakefulness stages, namely "wakefulness", "non-REM sleep", or "REM sleep" corresponding to each epoch of the training electroencephalogram data A.

When the training data A1000 includes only the training electromyogram data K, the label data Fe has a ground truth label indicating one of the sleep/wakefulness stages, namely "wakefulness", "non-REM sleep", or "REM sleep" corresponding to each epoch of the training electromyogram data K.

When the training data A1000 includes both of the training electroencephalogram data A and the training electromyogram data K, the label data Fe has a ground truth label indicating one of the sleep/wakefulness stages, namely "wakefulness", "non-REM sleep", or "REM sleep" corresponding to each epoch common to both of the training electroencephalogram data A and the training electromyogram data K.

Note that in the machine learning processing of the machine learning model 210, the machine learning unit 200 may use all of the entropy D1000, the entropy D1100, and the analysis data D1200, or may select and use some of the entropy D1000, the entropy D1100, and the analysis data D1200.

When generating the learning data used for the machine learning of the machine learning model 210, the machine learning unit 200 may expand the learning data by multiple magnitude rescaling methods (multiple_rescaling) such as standardization or normalization using a plurality of thresholds. The machine learning unit 200 may use only the standardization, may use only the normalization using the plurality of thresholds, or may use both of the standardization and the normalization using the plurality of thresholds.

The machine learning model 210 trained by the machine learning unit 200 is used as a determination model 210e in a sleep/wakefulness stages determination device 2e.

Note that in the configuration example in FIG. 9, the information processing device 1e includes the amplitude and phase entropy processing unit 1000, the narrowband entropy processing unit 1100, and the analysis processing unit 1200. However, the information processing device 1e may include only one or two of the amplitude and phase entropy processing unit 1000, the narrowband entropy processing unit 1100, and the analysis processing unit 1200.

### Sleep/Wakefulness Stages Determination Device

The sleep/wakefulness stages determination device 2e according to the fifth embodiment will be described with reference to FIG. 10. The sleep/wakefulness stages determination device 2e receives determination data A2000, which is a subject for determining the sleep/wakefulness stages. The determination data A2000 corresponds to the training data A1000 used by the information processing device 1e. When the training data A1000 includes only the training electroencephalogram data A, the determination data A2000 includes only electroencephalogram data G. When the training data A1000 includes only the training electromyogram data K, the determination data A2000 includes only electromyogram data P. When the training data A1000 includes both of the training electroencephalogram data A and the training electromyogram data K, the determination data A2000 includes both of the electroencephalogram data G and the electromyogram data P. The electroencephalogram data G may be data obtained by spline interpolation.

The sleep/wakefulness stages determination device 2e includes an amplitude and phase entropy processing unit 1000, a narrowband entropy processing unit 1100, an analysis processing unit 1200, a determination unit 300, and a post-determination processing unit 2000. The amplitude and phase entropy processing unit 1000, the narrowband entropy processing unit 1100, and the analysis processing unit 1200 are the same as the units 1000, 1100, and 1200 of the information processing device 1e in FIG. 9 described above, and descriptions thereof will be omitted.

In the sleep/wakefulness stages determination device 2e, the amplitude and phase entropy processing unit 1000 transmits an entropy D1000, which is a result of the entropy analysis on the determination data A2000, to the determination unit 300.

In the sleep/wakefulness stages determination device 2e, the narrowband entropy processing unit 1100 transmits an entropy D1100, which is a result of the entropy analysis on the determination data A2000, to the determination unit 300.

In the sleep/wakefulness stages determination device 2e, the analysis processing unit 1200 transmits analysis data D1200, which is a result of the analysis of the determination data A2000, to the determination unit 300.

The determination unit 300 determines the sleep/wakefulness stages (wakefulness, non-REM sleep, REM sleep) from the entropy D1000, the entropy D1100, and the analysis data D1200 using the determination model 210e. The determination unit 300 outputs a sleep/wakefulness stages determination result H. The sleep/wakefulness stages determination result H may be information that specifies one of "wakefulness", "non-REM sleep", or "REM sleep", or may be confidence of each of "wakefulness", "non-REM sleep", and "REM sleep".

The post-determination processing unit 2000 includes an RSE method execution unit 2100 and a skipping unit 2200.

The RSE method execution unit 2100 applies the RSE method to the sleep/wakefulness stages determination result H, which is the determination result of the sleep/wakefulness stages (wakefulness, non-REM sleep, REM sleep) determined using the determination model 210e, and performs processing of eliminating epochs having short stage durations by lowering priority of epochs determined to have stage durations shorter than a certain time, starting from an epoch having the shortest stage duration, in the sleep/wakefulness stages determination results H.

The skipping unit 2200 performs processing to suppress display of epochs with a sufficiently high degree of confidence among the sleep/wakefulness stages determination results H, which are determination results of sleep/wakefulness stages (wakefulness, non-REM sleep, REM sleep) determined using the determination model 210e.

The post-determination processing unit 2000 outputs determination output data H2000, which is a result of the processing by the RSE method execution unit 2100 and the skipping unit 2200.

Note that in the configuration example in FIG. 10, the post-determination processing unit 2000 includes both of the RSE method execution unit 2100 and the skipping unit 2200. However, the post-determination processing unit 2000 may include only one of the RSE method execution unit 2100 or the skipping unit 2200.

The post-determination processing unit 2000 is not essential, and the sleep/wakefulness stages determination device 2e does not necessarily need to include the post-determination processing unit 2000.

The above is a description of the fifth embodiment.

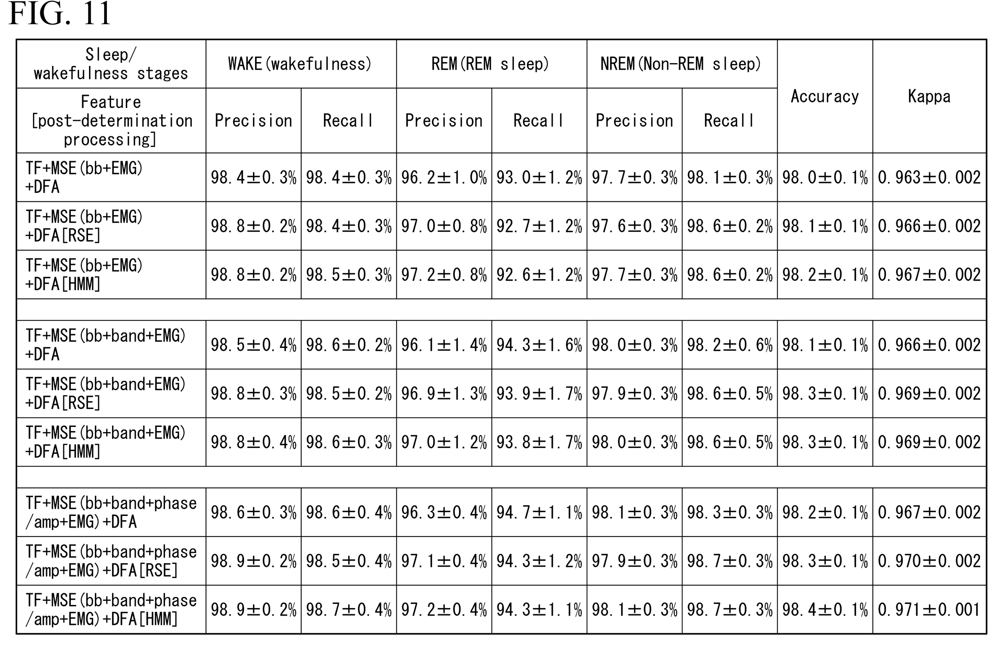

FIGS. 11, 12, and 13 are tables showing evaluation results according to the present disclosure. The evaluation results in FIGS. 11, 12, and 13 are analysis results by "5-fold cross-validation".

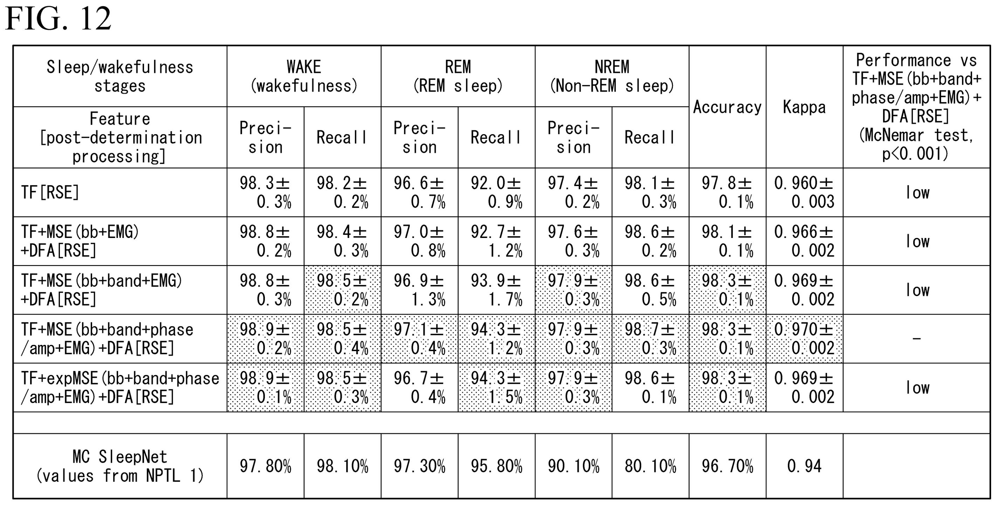

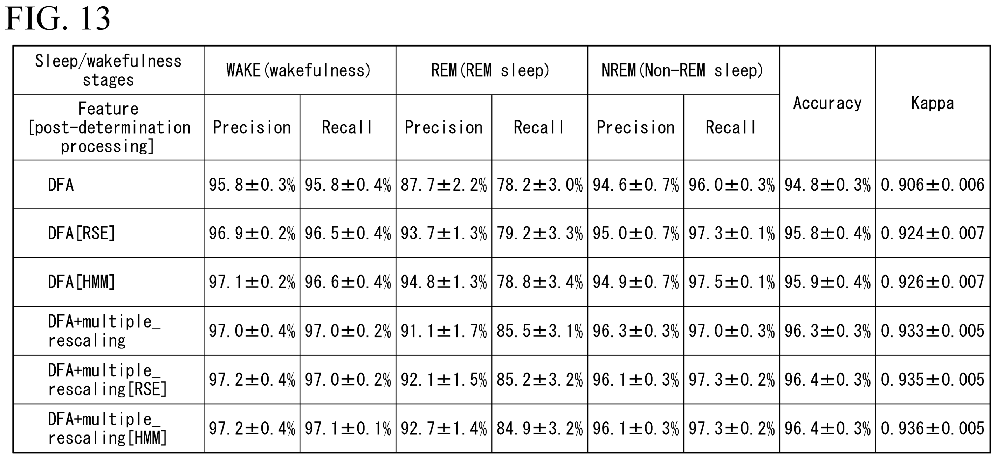

Note that, for an item of "MC SleepNet" in FIG. 12, numerical values are quoted from NPTL 1 for comparison with the present disclosure. Blank spaces in FIG. 13 indicate that an evaluation had not been performed at the time of filing this application.

Terms shown in FIGS. 11, 12, and 13 will be described.
"TF" refers to a case where time-frequency analysis is performed in the analysis processing unit 1200. In a case of TF alone, a model is similar to SPINDLE described in NPTL 5.
"MSE(x)" refers to a case where multiscale entropy analysis is performed on time-series data of x.
"bb" refers to a broadband electroencephalogram.
"band" refers to a narrowband electroencephalogram.
"phase/amp" refers to phase/amplitude of the narrowband electroencephalogram.
"EMG" refers to an electromyogram.
"DFA" refers to detrended fractal analysis.
"expMSE(x)" refers to a case where expanded entropy analysis is performed on the time-series data of x.
"MC SleepNet" refers to a model described in NPTL 1.
"multiple_rescaling" refers to "multiple magnitude rescaling methods".
"[RSE]" refers to a case where the RSE method is executed in the post-determination processing unit 2000.
"[HMM]" refers to a case where a hidden Markov model similar to that in NPTL 5 was executed in the post-determination processing unit 2000 instead of the RSE method.
"Precision" refers to a precision rate.
"Recall" refers to a recall rate.
"Accuracy" refers to an accuracy rate.
"Kappa" refers to a Cohen's kappa coefficient.
"Performance vs TF + MSE(bb + band + phase/amp + EMG) + DFA[RSE] (McNemar test, p < 0.001)" indicates that when correctness of the model corresponding to that row is compared to correctness of the model using "TF + MSE(bb + band + phase/amp + EMG) + DFA[RSE]", which is one aspect of the present disclosure, using the McNemar test, a result of determining whether the performance of this model is higher or lower than "TF + MSE(bb + band + phase/amp + EMG) + DFA[RSE]" with "p < 0.001" as a threshold.

The evaluation results in FIGS. 11, 12, and 13 show that the accuracy of determining the sleep/wakefulness stages according to the present disclosure is improved as a whole, as indicated, for example, by shaded areas in FIG. 12. In particular, significant improvement in the accuracy of determining REM (REM sleep), which has been difficult in the past, is a remarkable effect of the sleep/wakefulness stages determination according to the present disclosure.

The evaluation results in FIG. 12 show that as the number of features is increased by decomposing the entropy analysis from "broadband" to "broadband + narrowband" to "broadband + narrowband + amplitude/phase", the accuracy of determining the sleep/wakefulness stages is gradually improved as a whole (significant according to the McNemar test).

As shown in, for example, NPTL 1, in the past, there has been a problem in that the accuracy of determining REM is difficult to improve because the sleep/wakefulness stages are biased (with less REM), and improving the accuracy of determining WAKE (wakefulness) and NREM (non-REM sleep) is more advantageous for improving the overall accuracy in the sleep/wakefulness stages determination. However, according to the method of the present disclosure, as shown in the evaluation results in FIG. 12, an overall accuracy improvement of 0.5% is achieved compared to the SPINDLE-like model, and a significant improvement of 2.3% is achieved in the evaluation of Recall for REM.

As shown in the evaluation results in FIG. 12, even with a model with reduced computational load (a model accelerated by seven times or more by calculating expMSE using only a 1-second portion at the center of the 25-second window), nearly equivalent accuracy of determination was achieved as with the model using MSE.

A computer program for implementing the functions of the devices described above may be recorded in a computer-readable recording medium, and the program recorded in this recording medium may be read and executed by a computer system to implement the functions. Note that "computer system" as used here may include hardware such as an OS and peripheral devices.

Further, "computer-readable recording medium" refers to a storage device such as a writable non-volatile memory such as a flexible disk, a magneto-optical disk, a ROM, and a flush memory; a portable medium such as a digital versatile disk (DVD); and a hard disk built into computer system.

Furthermore, "computer-readable recording medium" includes a medium that holds a program for a certain period of time, such as a volatile memory (e.g. dynamic random access memory (DRAM)) inside a server or a client computer system when the program is transmitted via a network such as the Internet or a communication line such as a telephone line.

In addition, the program may be transmitted from a computer system that stores the program in a storage device or the like, to another computer system via a transmission medium, or by a transmission wave in the transmission medium. Here, "transmission medium" for transmitting a program refers to a medium that has a function of transmitting information, such as a network (communication network) such as the Internet or a communication line (communication cable) such as a telephone line.

The program described above may be configured to implement some of the functions described above. Furthermore, the functions described above may be implemented in combination with a program already recorded in the computer system, that is, the program may be a so-called differential file (differential program).

Technical matters according to the present disclosure will be described below.

### Time Series Analysis

The experimental data according to the present disclosure were measured under various conditions, so the conditions were standardized through preprocessing. The electroencephalogram (EEG) and electromyogram (EMG) signals were subjected to bandpass filtering from 0.5 to 50 Hz, and converted into a time series with a sampling frequency of 128 Hz using cubic spline interpolation. This time series is referred to as a broadband (bb) time series. These signals were decomposed into different frequency bands using finite impulse response filters: 2 to 4 Hz (delta), 4 to 8 Hz (theta), 8 to 13 Hz (alpha), 13 to 25 Hz (beta), and 25 to 45 Hz (gamma). This time series was divided into 25-second epochs using a 1-second sliding window.

Next, irregularity and unpredictability were quantified using sample entropy (SampEn), which is an example of entropy analysis, and fractal properties were quantified using detrended fractal analysis (DFA). Regarding these complexity analyses, typically, an embedding dimension m = 2 and a tolerance r of 0.1 to 0.25 SD of a standard deviation of the original time series before coarse-graining are often chosen to calculate SampEn. For investigating oscillatory profiles, a value of r = 0.2 SD was used, while for machine learning, a feature set was extended using three different parameters of r = 0.1 SD, 0.2 SD, and 0.4 SD. Further, for multi-scale entropy (MSE), that is, a profile of SampEn across multiple time scales, scale factors = 1, 2, 4, 8, 16, 32, and 64 were selected, with corresponding time delays set to 7.8, 15.6, 31.3, 62.5, 125, 250, and 500 ms. To ensure accuracy, it is recommended to use at least several hundred data points for SampEn calculations. Typically, coarse-graining is used in MSE calculations, but coarse-graining results in loss of data points as the time scale increases. For example, when performing entropy analysis on one second of data with a sampling frequency of 100 Hz, there are 100 data points when viewed at a time scale of 0.01 seconds, but only 50 points at a time scale of 0.02 seconds and only 25 points at a time scale of 0.04 seconds. Thus, coarse-graining reduces the number of data points used for calculating entropy, making it more susceptible to noise. Therefore, calculation using a sliding window preserves the data points, and further averaging within the window stabilizes the values. Averaging with a sliding window, that is, using a moving average. Thus, 3200 data points (128 Hz × 25 seconds) were used for the calculation of SampEn, independent of scale. Furthermore, in addition to the SampEn of bb and frequency-decomposed time series, the SampEn of the phase modulation and amplitude modulation in the narrow bands (delta to gamma bands) (phase and amplitude MSE) were also investigated. To avoid discontinuities in the time series, the phase SampEn was calculated for the cosine of the phase time series. Furthermore, the DFA was calculated for the standardized bb and narrowband time series amplitudes at the time windows n = 3, 4, 7, 11, 18, 29, 46, 73, 116, 184, 290, 459, 726, and 1147 (corresponding time scales = 23, 31, 55, 86, 141, 227, 359, 570, 906, 1438, 2266, 3586, 5672, and 8961 ms). Since each narrowband amplitude exhibited two different scaling regions, exponent values α thereof were investigated. The SampEn and DFA described here were calculated using the Python module AntroPy.

In addition, following the methods used in SPINDLE, EEG spectra and EMG amplitudes were used for machine learning applications. For EEG, a fast Fourier transform (FFT) was performed on the bb data from 2-second epochs, and adjacent frequencies were averaged to create 24 frequencies (1 to 24 Hz) in 1 Hz steps, prepared with a 0.125-second sliding window. For EMG, FFT was applied to 2-second epochs of EMG data, and frequencies from 0.5 to 30 Hz were integrated, repeating 24 times to match the EEG. The logarithms of these values were standardized over 24 hours, creating an input matrix of 24 frequencies × 160 time points × EEG/EMG for each epoch.

### Machine Learning

To automate sleep scoring, machine learning techniques were applied utilizing complexity features. Data from a total of 72 WT mice were used in the training, verification, and testing phases. The sleep/wakefulness stages in the machine learning dataset were scored by a single expert. Each sleep stage required a minimum duration of 12 seconds, and stages shorter than this threshold were adjusted to match the preceding or following stages.

A SAC model comprises three convolution blocks for feature extraction from time-frequency analysis (TF), MSE, and DFA, with outputs thereof integrated through fully connected layers. The TF analysis followed the SPINDLE algorithm, processing input of 24 frequencies × 160 time points × EEG/EMG through a convolution block including a max-pooling layer with filter size and stride of (2, 3), a convolution layer with 50 filters, filter size of (3, 3) and stride of (1, 1), and another max-pooling layer with filter size and stride of (2, 2). In the original SPINDLE experiment, two EEG channels and one EMG channel were used, but in the experiment according to the present disclosure, the model was trained with one EEG channel and one EMG channel. On the other hand, for MSE and DFA, four-dimensional inputs were prepared using multiple parameters and magnitude rescaling. MSE analyses were performed on 17 types of time series including bb, EMG, and five narrow bands, each decomposed into three types (normal/phase/amplitude), using seven time scales and three tolerance levels. DFA was applied to seven time series (bb, five narrowband amplitudes, and EMG) across 14 time scales. Various magnitude rescaling methods were applied, and for all mice, standardization was performed after excluding 1%, 3%, 5%, 7%, and 9% percentile edges within each mouse, resulting in seven magnitude rescaled inputs (typically, the optimal one of these is often used, but considering that the optimal rescaling criterion may differ depending on the features, and due to benefits of data augmentation, all these rescaling results were used as inputs for machine learning). Similar to the TF block, dimensions of EMG were matched with dimensions of EEG for concatenation. These procedures resulted in four-dimensional matrices with MSE inputs comprising 16 time series × 7 time scales × 7 magnitude rescales × 6 (3 tolerances × EEG/EMG), and DFA inputs comprised 6 time series × 14 time scales × 7 magnitude rescales × EEG/EMG. Local features were extracted using convolution layers with 50 filters for MSE and 30 filters for DFA, filter size of (2, 2, 2), and stride of (1, 1, 1). The features extracted from the TF, MSE, and DFA blocks were integrated through two layers of fully connected networks implementing 50% dropout and batch normalization (with 1000 nodes in a first layer and 500 nodes in a second layer), and then output via a softmax function for the WAKE, REM, and NREM stages. In contrast, the SPINDLE model integrated features through two fully connected layers (each with 1000 nodes) that implemented only 50% dropout.

In the experiments related to SPINDLE, an HMM algorithm that takes into account transition probabilities was used to improve prediction results using a convolutional neural network (CNN). However, this method may fail to detect features specific to mice with abnormal sleep architecture, such as OXKO mice (e.g., SOREM). To counteract this, the Rescoring Short Epoch (RSE) method was introduced as a constraint technique, replacing stages shorter than 12 seconds with the next most probable stage (i.e., the stage with the next highest softmax value) to match expert scoring rules (minimum duration of 12 seconds for each stage). The REM stage, which has the shortest duration among the sleep/wakefulness stages often lasts for several tens of seconds, so the threshold of 12 seconds is considered reasonable.

The invention described in the claims originally attached to Japanese Patent Application No. 2023-111974 will be appended below. The numbering of supplementary notes is the same as that in the claims originally attached to Japanese Patent Application No. 2023-111974.

### Supplementary Note 1

An information processing device including:
an electroencephalogram processing unit configured to obtain an amplitude and a phase from training electroencephalogram data measured from a living body being a mammal for a plurality of frequency bands according to individual frequency bands, the plurality of frequency bands being predetermined, and perform complexity analysis processing comprising entropy analysis on each of the amplitude and the phase obtained in each of the plurality of frequency bands; and
a machine learning unit configured to perform machine learning processing of a machine learning model configured to determine sleep/wakefulness stages using a result of the complexity analysis processing and label data of the sleep/wakefulness stages corresponding to the training electroencephalogram data.

### Supplementary Note 2

The information processing device according to supplementary note 1, in which
the electroencephalogram processing unit further extracts signals in the plurality of frequency bands, separately, from the training electroencephalogram data, and performs complexity analysis processing comprising entropy analysis on each of the signals extracted in each of the plurality of frequency bands, and
the machine learning unit performs machine learning processing of the machine learning model by further using a result of the complexity analysis processing.

### Supplementary Note 3

The information processing device according to supplementary note 2, in which
the electroencephalogram processing unit further performs complexity analysis processing comprising entropy analysis on the training electroencephalogram data, and
the machine learning unit performs machine learning processing of the machine learning model by further using a result of the complexity analysis processing.

### Supplementary Note 4

The information processing device according to supplementary note 1, in which
the electroencephalogram processing unit further performs complexity analysis processing comprising fractal analysis on the amplitude in each of the plurality of frequency bands, and
the machine learning unit performs machine learning processing of the machine learning model by further using a result of the complexity analysis processing.

### Supplementary Note 5

The information processing device according to supplementary note 4, in which
the electroencephalogram processing unit further extracts signals in the plurality of frequency bands, separately, from the training electroencephalogram data, and performs complexity analysis processing comprising entropy analysis on each of the signals extracted in each of the plurality of frequency bands, and
the machine learning unit performs machine learning processing of the machine learning model by further using a result of the complexity analysis processing.

### Supplementary Note 6

The information processing device according to supplementary note 5, in which
the electroencephalogram processing unit further performs complexity analysis processing comprising entropy analysis and fractal analysis on the training electroencephalogram data, and
the machine learning unit performs machine learning processing of the machine learning model by further using a result of the complexity analysis processing.

### Supplementary Note 7

The information processing device according to any one of supplementary notes 1 to 6, further including a frequency analysis unit configured to convert the training electroencephalogram data into a frequency spectrum, in which the machine learning unit performs machine learning processing of the machine learning model further using the frequency spectrum converted from the training electroencephalogram data.

### Supplementary Note 8

The information processing device according to supplementary note 7, further including an amplitude analysis unit configured to obtain an amplitude from training electromyogram data measured from the living body,
in which
the machine learning unit performs machine learning processing of the machine learning model by further using the amplitude obtained from the training electromyogram data, and
the label data is label data of sleep/wakefulness stages corresponding to the training electromyogram data.

### Supplementary Note 9

The information processing device according to supplementary note 8, further including a complexity analysis unit configured to perform complexity analysis processing comprising entropy analysis and fractal analysis on the training electromyogram data,
in which the machine learning unit performs machine learning processing of the machine learning model by further using a result of the complexity analysis processing on the training electromyogram data.

### Supplementary Note 10

The information processing device according to any one of supplementary notes 1 to 6, in which the plurality of frequency bands are a delta wave band, a theta wave band, an alpha wave band, a beta wave band, and a gamma wave band.

### Supplementary Note 11

The information processing device according to any one of supplementary notes 1 to 6, the living body is a mouse.

### Supplementary Note 12

A sleep/wakefulness stages determination device including:
an electroencephalogram processing unit configured to obtain an amplitude and a phase from electroencephalogram data measured from a living body being a mammal for a plurality of frequency bands according to individual frequency bands, the plurality of frequency bands being predetermined, and perform complexity analysis processing comprising entropy analysis on each of the amplitude and the phase obtained in each of the plurality of frequency bands; and
a determination unit configured to determine sleep/wakefulness stages from a result of the complexity analysis processing using a determination model.

### Supplementary Note 13

The sleep/wakefulness stages determination device according to supplementary note 12, in which
the electroencephalogram processing unit further extracts signals in the plurality of frequency bands, separately, from the electroencephalogram data, and performs complexity analysis processing comprising entropy analysis on each of the signals extracted in each of the plurality of frequency bands, and
the determination unit determines the sleep/wakefulness stages by further using a result of the complexity analysis processing.

### Supplementary Note 14

The sleep/wakefulness stages determination device according to supplementary note 13, in which
the electroencephalogram processing unit further performs complexity analysis processing comprising entropy analysis on the electroencephalogram data, and
the determination unit determines the sleep/wakefulness stages by further using a result of the complexity analysis processing.

### Supplementary Note 15

The sleep/wakefulness stages determination device according to supplementary note 12, in which
the electroencephalogram processing unit further performs complexity analysis processing comprising fractal analysis on the amplitude in each of the plurality of frequency bands, and
the determination unit determines the sleep/wakefulness stages by further using a result of the complexity analysis processing.

### Supplementary Note 16

The sleep/wakefulness stages determination device according to supplementary note 15, in which
the electroencephalogram processing unit further extracts signals in the plurality of frequency bands, separately, from the electroencephalogram data, and performs complexity analysis processing comprising entropy analysis on each of the signals extracted in each of the plurality of frequency bands, and
the determination unit determines the sleep/wakefulness stages by further using a result of the complexity analysis processing.

### Supplementary Note 17

The sleep/wakefulness stages determination device according to supplementary note 16, in which
the electroencephalogram processing unit further performs complexity analysis processing comprising entropy analysis and fractal analysis on the electroencephalogram data, and
the determination unit determines the sleep/wakefulness stages by further using a result of the complexity analysis processing.

### Supplementary Note 18

The sleep/wakefulness stages determination device according to any one of supplementary notes 12 to 17, further including a frequency analysis unit configured to convert the electroencephalogram data into a frequency spectrum,
in which the determination unit determines the sleep/wakefulness stages further using the frequency spectrum converted from the electroencephalogram data.

### Supplementary Note 19

The sleep/wakefulness stages determination device according to supplementary note 18, further including an amplitude analysis unit configured to obtain an amplitude from electromyogram data measured from the living body,
in which the determination unit determines the sleep/wakefulness stages further using the amplitude obtained from the electromyogram data.

### Supplementary Note 20

The sleep/wakefulness stages determination device according to supplementary note 19, further including a complexity analysis unit configured to perform complexity analysis processing comprising entropy analysis and fractal analysis on the electromyogram data,
in which the determination unit determines the sleep/wakefulness stages by further using a result of complexity analysis processing on the electromyogram data.

### Supplementary Note 21

The sleep/wakefulness stages determination device according to any one of supplementary notes 12 to 17, in which the plurality of frequency bands are a delta wave band, a theta wave band, an alpha wave band, a beta wave band, and a gamma wave band.

### Supplementary Note 22

The sleep/wakefulness stages determination device according to any one of supplementary notes 12 to 17, in which the living body is a mouse.

### Supplementary Note 23

A computer program for causing a computer to execute:
obtaining an amplitude and a phase from training electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands; and
performing machine learning processing of a machine learning model for determination of sleep/wakefulness stages using a result of the complexity analysis processing and label data of the sleep/wakefulness stages corresponding to the training electroencephalogram data.

### Supplementary Note 24

A computer program for causing a computer to execute:
obtaining an amplitude and a phase from training electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands; and
determining sleep/wakefulness stages from a result of the complexity analysis processing using a determination model.

### Supplementary Note 25

An information processing method executed by an information processing device, the information processing method including:
obtaining an amplitude and a phase from training electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands; and
performing machine learning processing of a machine learning model for determination of sleep/wakefulness stages using a result of the complexity analysis processing and label data of the sleep/wakefulness stages corresponding to the training electroencephalogram data.

### Supplementary Note 26

A sleep/wakefulness stages determination method executed by a sleep/wakefulness stages determination device, the sleep/wakefulness stages determination method including:
obtaining an amplitude and a phase from electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands; and
determining sleep/wakefulness stages from a result of the complexity analysis processing using a determination model.

### Reference Sings List

1a, 1b, 1c, 1d Information processing device, 100 Electroencephalogram processing unit, 200 Machine learning unit, 110 Bandpass filter, 120 Hilbert transform unit, 130, 510 Complexity analysis unit, 131, 511 Entropy analysis unit, 132, 512 Fractal analysis unit, 210 Machine learning model, 2a, 2b, 2c, 2d Sleep/wakefulness stages determination device, 300 Determination unit, 210a, 210b, 210c, 210d, 210e Determination model, 400 Frequency analysis unit, 500 Amplitude analysis unit, 1000 Amplitude and phase entropy processing unit, 1100 Narrowband entropy processing unit, 1200 Analysis processing unit, 2000 Post-determination processing unit, 2100 RSE method execution unit, 2200 Skipping unit

## Claims

1. An information processing device comprising:
an electroencephalogram processing unit configured to obtain an amplitude and a phase from training electroencephalogram data measured from a living body being a mammal for a plurality of frequency bands according to individual frequency bands, the plurality of frequency bands being predetermined, and perform complexity analysis processing comprising entropy analysis on each of the amplitude and the phase obtained in each of the plurality of frequency bands; and
a machine learning unit configured to perform machine learning processing of a machine learning model configured to determine sleep/wakefulness stages using a result of the complexity analysis processing and label data of the sleep/wakefulness stages corresponding to the training electroencephalogram data.

2. The information processing device according to claim 1, wherein
the electroencephalogram processing unit further extracts signals in the plurality of frequency bands, separately, from the training electroencephalogram data, and performs complexity analysis processing comprising of entropy analysis on each of the signals extracted in each of the plurality of frequency bands, and
the machine learning unit performs machine learning processing of the machine learning model by further using a result of the complexity analysis processing.

3. The information processing device according to claim 1, wherein the entropy analysis on the phase calculates an entropy of a cosine of the phase by performing entropy analysis on the cosine of the phase.

4. The information processing device according to claim 1, wherein the electroencephalogram processing unit uses expanded sample entropy as entropy analysis.

5. The information processing device according to claim 1, wherein when generating learning data used for machine learning of the machine learning model, the machine learning unit expands the learning data by standardization or normalization using a plurality of thresholds, or by both standardization and normalization using a plurality of thresholds.

6. The information processing device according to any one of claims 1 to 5, wherein the living body is a mouse.

7. A sleep/wakefulness stages determination device comprising:
an electroencephalogram processing unit configured to obtain an amplitude and a phase from electroencephalogram data measured from a living body being a mammal for a plurality of frequency bands according to individual frequency bands, the plurality of frequency bands being predetermined, and perform complexity analysis processing comprising of entropy analysis on each of the amplitude and the phase obtained in each of the plurality of frequency bands; and
a determination unit configured to determine sleep/wakefulness stages from a result of the complexity analysis processing using a determination model.

8. The sleep/wakefulness stages determination device according to claim 7, wherein
the electroencephalogram processing unit further extracts signals in the plurality of frequency bands, separately, from the electroencephalogram data, and performs complexity analysis processing comprising of entropy analysis on each of the signals extracted in each of the plurality of frequency bands, and
the determination unit determines the sleep/wakefulness stages by further using a result of the complexity analysis processing.

9. The sleep/wakefulness stages determination device according to claim 7, wherein the entropy analysis on the phase calculates an entropy of a cosine of the phase by performing entropy analysis on the cosine of the phase.

10. The sleep/wakefulness stages determination device according to claim 7, wherein the electroencephalogram processing unit uses expanded sample entropy as entropy analysis.

11. The sleep/wakefulness stages determination device according to claim 7, further comprising a rescoring short epoch (RSE) method execution unit configured to apply an RSE method to determination results of the sleep/wakefulness stages determined using the determination model to eliminate epochs having short stage durations in the determination results of the sleep/wakefulness stages by lowering priority of epochs determined to have stage durations shorter than a certain time, starting from an epoch having the shortest stage duration.

12. The sleep/wakefulness stages determination device according to claim 7, further comprising a skipping unit configured to suppress display of an epoch with a sufficiently high degree of confidence in the determination results of the sleep/wakefulness stages determined using the determination model.

13. The sleep/wakefulness stages determination device according to claim 7, further comprising an accuracy display control unit configured to display accuracy of determination for the determination results of the sleep/wakefulness stages determined using the determination model.

14. The sleep/wakefulness stages determination device according to any one of claims 7 to 13, wherein the living body is a mouse.

15. A computer program for causing a computer to execute:
obtaining an amplitude and a phase from training electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands; and
performing machine learning processing of a machine learning model for determination of sleep/wakefulness stages using a result of the complexity analysis processing and label data of the sleep/wakefulness stages corresponding to the training electroencephalogram data.

16. A computer program for causing a computer to execute:
obtaining an amplitude and a phase from electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands; and
determining sleep/wakefulness stages from a result of the complexity analysis processing using a determination model.

17. An information processing method executed by an information processing device, the information processing method comprising:
obtaining an amplitude and a phase from training electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands; and
performing machine learning processing of a machine learning model for determination of sleep/wakefulness stages using a result of the complexity analysis processing and label data of the sleep/wakefulness stages corresponding to the training electroencephalogram data.

18. A sleep/wakefulness stages determination method executed by a sleep/wakefulness stages determination device, the sleep/wakefulness stages determination method comprising:
obtaining an amplitude and a phase from electroencephalogram data measured from a living body being a mammal for a plurality of frequency bands according to individual frequency bands, the plurality of frequency bands being predetermined, and performing complexity analysis processing comprising entropy analysis on each of the amplitude and the phase obtained in each of the plurality of frequency bands; and
determining sleep/wakefulness stages from a result of the complexity analysis processing using a determination model.
